# EUROPEAN PATENT SPECIFICATION

(11) **EP 2 695 617 B1**
(45) Date of publication and mention of the grant of the patent: **09.01.2019**
(21) Application number: 13174890.7
(22) Date of filing: 23.12.2010
(51) Int. Cl.: A61K 31/592, A61K 31/593, A61K 31/7032, A61K 31/7034, A61K 45/06, A61P 35/00, A61P 37/02

(54) **Vitamin D glycosides and sulfates for treating intestinal diseases**
Vitamin D Glycoside und Sulfate zur Behandlung von Darmerkrankungen
Utilisation de glycosides et de sulfates de vitamine D pour le traitement des maladies intestinales

(30) Priority: 23.12.2009 US 289789 P
(43) Date of publication of application: 12.02.2014
(62) Divisional of application: 10798695.2
(73) Proprietor: Glycomyr Inc., Ames, IA 50010 (US)
(72) Inventor: Goff, Jesse, Ames, IA 50010 (US); Horst, Ronald, Ames, IA 50014 (US)
(74) Representative: Ellis, Katherine Elizabeth Sleigh

(56) References cited:
- WO-A1-01/22974
- WO-A2-01/30751
- WO-A2-2008/057363
- JP-A- 58 216 178
- JP-A- 2009 247 339
- US-A- 4 410 515
- US-A- 4 521 410
- MIZUSHINA YOSHIYUKI ET AL: "Selective inhibition of mammalian DNA polymerase alpha by vitamin D2 and D3", JOURNAL OF PHARMACOLOGICAL SCIENCES, JAPANESE PHARMACOLOGICAL SOCIETY, TOKYO, JP, vol. 92, no. 3, 1 July 2003 (2003-07-01), pages 283-290, XP008133488, ISSN: 1347-8613
- Timothy A. Reinhardt ET AL: "Self-induction of 1,25-Dihydroxyvitamin D3 Metabolism Limits Receptor Occupancy and Target Tissue Responsiveness*", THE JOURNAL OF BIOLOGICAL CHEMISTRY, vol. 264, 25 September 1989 (1989-09-25), pages 15917-15921, XP055308335,

### SEQUENCE LISTING

<110> Glycomyr Inc.
<120> Use of Vitamin D Glycosides for Treatment of Disease
<130> KE/N30245
<150> 61/289,789
   <151> 2009-12-23
<160> 4
<170> PatentIn version 3.5
<210> 1
   <211> 20
   <212> DNA
   <213> Artificial Sequence
<220>
   <223> Cyp24-For
<400> 1
   cacacgctgg cctgggacac 20
<210> 2
   <211> 20
   <212> DNA
   <213> Artificial Sequence
<220>
   <223> Cyp24-Rev
<400> 2
   ggagctccgt gacagcagcg 20
<210> 3
   <211> 25
   <212> DNA
   <213> Artificial Sequence
<220>
   <223> GAPDH-For
<400> 3
   gaaggtcggt gtgaacggat ttggc 25
<210> 4
   <211> 26
   <212> DNA
   <213> Artificial Sequence
<220>
   <223> GAPDH-Rev
<400> 4
   ttgatgttag tggggtctcg ctcctg 26

## Description

This invention concerns modified vitamin D compounds, specifically glycosides and sulfates of vitamin D drugs, for use in treating tumours, hyperproliferative/neoplastic disorders, infectious disease, autoimmune disorders, and inflammatory disorders as defined in the claims.

Vitamin D is a generic term for a family of secosteroids that have affinity for the vitamin D receptor (VDR) and are involved in the physiologic regulation of calcium and phosphate metabolism. Exposure to the sun and dietary intake are common sources of vitamin D. Two forms of vitamin D include vitamin D₃ and its analog vitamin D₂. Vitamin D₃ is synthesized in human skin from 7-dehydrocholesterol and ultraviolet light. Vitamin D₃ or vitamin D₂ can be ingested from the diet, for example, in fortified milk products. The vitamin D₃ and D₂ forms of vitamin D are not considered to have any substantial biological activity and must first be converted to their active forms to be biologically active.

In being converted to their active forms, vitamin D₂ and D₃ first undergo hydroxylation in the liver to 25-hydroxyvitamin D. They then undergo hydroxylation in the kidney to 1α,25-dihydroxycholecalciferol, also known as 1,25-dihydroxyvitamin D or calcitriol. It is 1,25-dihydroxyvitamin D that is the principal biologically active form of vitamin D. The biological production of this active form of the vitamin is tightly physiologically regulated.

The endocrine functions of 1,25-dihydroxyvitamin D primarily concern maintenance of blood calcium and phosphate concentrations. It maintains blood calcium levels within the normal range by regulating intestinal calcium absorption. When intestinal absorption is unable to maintain calcium homeostasis, 1,25-dihydroxyvitamin D mobilizes calcium from bones.

In addition to its effects on blood calcium, 1,25-dihydroxyvitamin D has also been shown to have effects on hyperproliferative disorders, infections, and immune function.

Regarding hyperproliferative disorders, in vitro assays using 1,25-dihydroxyvitamin D or its analogs have demonstrated anti-proliferative effects in cell lines derived from many malignancies including prostate, breast, colon, pancreas, and endometrial carcinomas, in addition to squamous cell carcinoma, myeloid leukemia, and retinoblastoma. Tissues derived from neoplasia involving lung, bone marrow, melanoma, and sarcomas of the soft tissues also appear to be amenable to treatment with 1,25-dihydroxyvitamin D. The presence of the VDR has been described in carcinomas of the prostate, breast, colon, lung, pancreas, endometrium, bladder, cervix, ovaries, squamous cell carcinoma, renal cell carcinoma, myeloid and lymphocytic leukemia, medullary thyroid carcinoma, melanoma, multiple myeloma, retinoblastoma, and sarcomas of the soft tissues and bone.

With respect to immune function, vitamin D compounds have been shown to modulate immune cell function. For example, vitamin D status has been linked to the development of a number of different type 1 helper T cell (Th1)-mediated autoimmune diseases. These include type 1 diabetes, multiple sclerosis, and inflammatory bowel diseases. The active form of vitamin D (1,25 dihydroxyvitamin D) has been shown to ameliorate the development of clinical signs and lesions in experimental models of these autoimmune diseases.

Th1-mediated immunity is critical for the ability of the host to mount a protective immune response to many different infectious diseases. Vitamin D appears to play a critical role in this response. For example, there is growing evidence that vitamin D deficiency and reduced sunlight exposure result in increased susceptibility to tuberculosis. In addition, vitamin D deficiency in mice results in increased replication of *Mycobacterium bovis.*

Promising treatments using vitamin D-related therapies in humans for many of the above conditions have been thwarted by development of hypercalcemia induced by systemic use of the hormonally active form of vitamin D (1,25-dihydroxyvitamin D) and its analogs. Vitamin D and its metabolic products are very potent calcemic agents that cause elevated blood calcium levels by stimulating intestinal calcium absorption and bone calcium resorption. Hypercalcemia is detrimental to the health of an individual as it leads to constipation, bone pain, kidney stones, depression, fatigue, anorexia, nausea, vomiting, pancreatitis, and increased urination among other problems. Hypercalcemia can be life-threatening.

Feeding potential subjects a low calcium diet prior to treatment with vitamin D compounds has been a recommended method for reducing the risk of development of symptomatic hypercalcemia. However, placing animals on a low-calcium diet reduces the number of vitamin D receptors in renal and intestinal tissue (Goff JP, Reinhardt TA, Beckman MJ, Horst RL. Endocrinology. 1990 Feb;126(2):1031-5) and increases the activity of 1,25-dihydroxyvitamin D-24-hydroxylase (24-hydroxylase) (Goff JP, Reinhardt TA, Engstrom GW, Horst RL. Endocrinology. 1992 Jul;131(1):101-4). The vitamin D 24-hydroxylase is involved in the breakdown of the active forms of vitamin D. Thus, placing subjects on a low-calcium diet prior to treatment with vitamin D compounds leads to mechanisms that reduce the effectiveness of the vitamin D treatment.

A need exists for methods of treating hyperproliferative disorders, immune function disorders, and infection through vitamin D-related pathways without inducing hypercalcemia.

The present invention relates to a pharmaceutical composition comprising a therapeutically effective and non-severe-symptomatic-hypercalcemia-inducing amount of a first compound and a second compound; wherein the first compound is a vitamin D prodrug comprising a vitamin D-drug moiety and a pro moiety, wherein the pro moiety is selected from the group consisting of a glycone moiety and a sulfate moiety, wherein the vitamin D prodrug has the structure of Formula (I) as defined in claim 1; wherein the second compound comprises a first moiety and a second moiety, wherein the first moiety is selected from the group consisting of a glycone moiety and a sulfate moiety, and wherein the second moiety is a 24-hydroxylase inhibitor selected from the group consisting of 25-hydroxyvitamin D2, 24,25-dihydroxyvitamin D2, 25-hydroxyvitamin D3, 24,25-dihydroxyvitamin D3, 25-hydroxyvitamin D4, 24,25-dihydroxyvitamin D4, 25-hydroxyvitamin D5, and 24,25-dihydroxyvitamin D5, for selectively treating a vitamin D-sensitive disease in the intestine as defined in the claims. Disclosed is a use in treating hyperproliferative, autoimmune, or infectious diseases by administering to a subject prodrugs of vitamin D and its analogs, *i.e*., "vitamin D drugs." The vitamin D prodrugs of the present invention include glycosides of vitamin D drugs and sulfates of vitamin D drugs. The vitamin D drugs are biologically inert until the glycosidic bond or the sulfate ester bond, respectively, is cleaved, releasing the vitamin D drug in the vicinity of the diseased tissues or cells. In some versions of the invention, tumours, bacteria, or cells contributing to autoimmune disease exhibit elevated levels of enzymes capable of cleaving the prodrugs and thereby free the vitamin D in their vicinity. In other versions of the invention, enzymes capable of cleaving the vitamin D prodrugs are targeted to the diseased tissues or cells. Treatment of subjects with the vitamin D prodrugs allows for the beneficial effects of vitamin D with respect to hyperproliferative, autoimmune, or infectious diseases without the hypercalcemia, which results from conventional vitamin D treatment.

Disclosed is a use in treating a vitamin D-sensitive disease selected from the group consisting of a hyperproliferative, autoimmune, or infectious disease without inducing severe symptomatic hypercalcemia. The method comprises administering to a patient suffering from the vitamin D-sensitive disease a therapeutically effective and non-severe-symptomatic-hypercalcemia-inducing amount of a vitamin D prodrug, wherein the administered vitamin D prodrug comprises a vitamin D-drug moiety and a pro moiety, and wherein the pro moiety is selected from the group consisting of a glycone moiety and a sulfate moiety.

Also disclosed is a use in a method of treating a vitamin D-sensitive intestinal disease without inducing severe symptomatic hypercalcemia. The method comprises administering to a patient suffering therefrom a therapeutically effective and non-severe-symptomatic-hypercalcemia-inducing amount of a vitamin D prodrug, wherein the administered vitamin D prodrug includes a vitamin D-drug moiety and a pro moiety, and wherein the pro moiety is selected from the group consisting of a glycone moiety and a sulfate moiety.

In a more specific version, the method comprises selectively treating the vitamin D-sensitive intestinal disease in the lower intestine. The method comprises activating the vitamin D prodrug in the lower intestine by cleaving the vitamin D-drug moiety from the pro moiety in the lower intestine.

The invention includes administering a first vitamin D prodrug and a second vitamin D prodrug as defined in the claims. The first vitamin D prodrug comprises an active vitamin D drug as a vitamin D-drug moiety. The second vitamin D prodrug comprises an inactive vitamin D drug as a vitamin D-drug moiety. The second vitamin D prodrug preferably potentiates a therapeutic effect of the first vitamin D prodrug. The potentiation may occur by inhibiting the turnover of the active vitamin D drug at a target site.

Other versions of the invention include pharmaceutical compositions or preparations for use in any of the methods described herein. One version is a composition comprising a first vitamin D prodrug or pharmaceutical salt thereof, and a second vitamin D prodrug or pharmaceutical salt thereof. The first vitamin D prodrug and the second vitamin D prodrug each comprises a vitamin D-drug moiety and a pro moiety. The vitamin D-drug moiety of the first vitamin D prodrug is an active vitamin D drug that is present in a therapeutically effective amount. The vitamin D-drug moiety of the second vitamin D prodrug is an inactive vitamin D drug that is present in an amount that potentiates effectiveness of the first vitamin D prodrug.

The objects and advantages of the invention will appear more fully from the following detailed description of the preferred embodiment of the invention.
FIG. 1A shows fold change in expression of the 25-hydroxyvitamin-D-24-hydroxylase enzyme (Cyp24) in the colon of mice orally administered increasing doses (6, 12, 24, or 48 pmol) of 1,25-dihydroxyvitamin D₃ (1,25D₃) or 1,25-dihydroxyvitamin D₃-25β-glucuronide (Gluc-1,25D₃) and sacrificed 6 hrs after treatment (N=4 mice/treatment).
FIG. 1B shows fold change in expression of Cyp24 in the duodenum of mice treated as described for FIG. 1A.
FIG. 1C shows plasma concentrations of 1,25-dihydroxyvitamin D in mice treated as described for FIG. 1A.
FIG. 2A shows fold change in expression of Cyp24 in the colon of mice orally administered 24 pmol of 1,25-dihydroxyvitamin D₃ (1,25D₃) or 1,25-dihydroxyvitamin D₃-25β-glucuronide (Gluc-1,25D₃) and sacrificed at 1, 3, 6, or 24 hrs after treatment (N=5 mice/treatment time point).
FIG. 2B shows fold change in expression of Cyp24 in the duodenum of mice treated as described for FIG. 2A.
FIG. 2C shows plasma concentrations of 1,25-dihydroxyvitamin D in mice treated as described for FIG. 2A.
FIG. 3 depicts 1,25-dihydroxyvitamin D₃-25β-glucuronide, a preferred vitamin D prodrug of the present invention.

"Vitamin D prodrug" refers to compounds having a vitamin D-drug moiety and a pro moiety. Vitamin D prodrugs described herein can be vitamin D glycosides or vitamin D sulfates. Vitamin D glycosides comprise a glycone moiety as the pro moiety, and vitamin D sulfates comprise a sulfate moiety as the pro moiety.

"Glycoside" refers to a molecule in which a sugar is bound to a non-carbohydrate moiety. The sugar component is termed the "glycone" moiety, and the non-carbohydrate component is termed the "aglycone" moiety. If the glycone group of a glycoside is glucose, then the molecule is termed a "glucoside"; if it is fructose, then the molecule is termed a "fructoside"; if it is glucuronic acid, then the molecule is termed a "glucuronide." Examples of glycone groups which can used in the present invention include galactosyl, glucuronyl, deoxy-glucosyl, iduronyl, glucosyl, N-acetyl glucosaminosyl, fructosyl, sialosyl, hyaluronosyl, sedoheptulosyl, xylulosyl, ribulosyl, ribosyl, xylitosyl, daunosaminosyl, arabinosyl, fucosyl, deoxy-ribosyl, mannosyl, N-acetyl-galactosyl, rhamnosyl, 3,6-anhydrogalactosyl, sialylfucosyl, and xylosyl. Other acceptable glycone groups are described elsewhere in this document. "Vitamin D glycoside" refers to a glycoside having a vitamin D-drug moiety as the aglycone moiety.

"Glycosidase" refers to a molecular species which is able to effect cleavage of a glycoside whereby the glycone moiety is cleaved from the aglycone moiety. Examples of glycosidases are glucuronidase, galactosidase, glucosidase, iduronidase, lysozyme, amylase, N-acetyl glucosaminidase, fructosidase, sialidase, hyaluronidase, etc., these being defined by the activity which each possess. Glycosidase activity is not a property solely of proteins. Synthetic chemistry can also generate similar activities (ability to hydrolyse the glycosidic bond). Examples of substrates for such glycosidases are lactose, glycogen, starch, cellulose, sucrose, nitrophenyl-maltohexoside, maltotriose, bromo-chloro-indolyl galactoside, methylumbelliferyl-N-acetylneuraminic acid, and nitrophenyl glucoside. Other acceptable glycosidases are described elsewhere in this document.

"Sulfatase" refers to a molecular species which is able to effect cleavage of a vitamin D sulfate, whereby the vitamin D-drug moiety is cleaved from the pro (*i.e*., sulfate) moiety.

Unless specifically implied to the contrary, "vitamin D" without a subscript, used alone, as a suffix or prefix, or as a modifier, refers to any of vitamin D₂ (ergocalciferol), vitamin D₃ (cholecalciferol), vitamin D₄ (22-dihydroergocalciferol), and vitamin D₅ (sitocalciferol).

"Vitamin D drug" refers to 1,25-dihydroxyvitamin D compounds (*i.e*., 1,25-dihydroxyvitamin D₂, 1,25-dihydroxyvitamin D₃, 1,25-dihydroxyvitamin D₄, and 1,25-dihydroxyvitamin D₅); active analogs thereof; or inactive analogs thereof that increase the blood, tissue, or cellular level of a 1,25-dihydroxyvitamin D compound or an active analog thereof.

"Analogs," used with reference to 1,25-dihydroxyvitamin D compounds, refers to biological precursors of 1,25-dihydroxyvitamin D compounds, biological metabolites of 1,25-dihydroxyvitamin D compounds, or any natural or synthetic compound recognized in the art as having a structural similarity to-or being derived from-1,25-dihydroxyvitamin D compounds. Analogs of 1,25-dihydroxyvitamin D₂ or 1,25-dihydroxyvitamin D₃ therefore include any of the family of secosteroids derived from vitamin D₂ (ergocalciferol), vitamin D₃ (cholecalciferol), vitamin D₄ (22-dihydroergocalciferol), and vitamin D₅ (sitocalciferol) or their metabolites or precursors such as ergosterol (7-dehydro-22-dehydro-24-methyl-cholesterol) and 7 dehydrocholesterol, 25-hydroxyvitainin D, the 3-hydroxylated dihydrotachysterol₂, and the 1α-hydroxylated alfacalcidol (1α-hydroxyvitamin D₃), as well as the numerous natural and synthetic vitamin D compounds defined elsewhere herein or described in Bouillon et. al, Endocrine Reviews. 1995 16:200-257.

"Active" used with reference to analogs of 1,25-dihydroxyvitamin D compounds refers to those analogs that directly produce a vitamin D-dependent effect in a target tissue or target cell without being modified or further metabolized by a non-target tissue, non-target cell, or other site elsewhere in the body. When used to modify "vitamin D drug," the term "active" refers to 1,25-dihydroxyvitamin D compounds or active analogs thereof. An "inactive" analog or vitamin D drug is one that is not active. "Vitamin D-dependent effects" include any of the effects disclosed herein, known in the art, or hereafter discovered that result from administration or treatment of 1,25-dihydroxyvitamin D compounds. Examples of various vitamin D-dependent effects are described throughout this document and include, without limitation, anti-proliferative effects, antirachitic effects, and immunomodulatory effects, particularly with respect to Th1-mediated diseases and bacterial infection. Active vitamin D drugs may elicit one or some but not necessarily all of the effects of 1,25-dihydroxyvitamin D compounds.

A subset of active vitamin D drugs includes those that have an affinity for the vitamin D receptor. "Vitamin D receptor" (or VDR) refers to a protein transcription factor, for which the gene and its product have already been characterized and found to contain 427 amino acids with a molecular weight of about 47,000, or variants thereof. The full length cDNA of the human VDR is disclosed in Baker et al., PNAS, USA. 1988 85:3294-3298. "Affinity for the vitamin D receptor" includes binding to the vitamin D receptor with a Relative Competitive Index (RCI) of 0.05 or greater or, more particularly, 5 or greater, including 5-250. The RCI is indexed to an RCI of 100 for calcitriol. It is preferred that a compound having an affinity for the vitamin D receptor is a vitamin D receptor agonist. Not all 1,25-dihydroxyvitamin D₃-dependent effects result from activating the vitamin D receptor. Therefore, another subset of active vitamin D drugs are those that produce vitamin D-dependent effects independently of the vitamin D receptor.

A subset of inactive vitamin D drugs that increase the blood, tissue, or cellular level of active vitamin D drugs include those that competitively inhibit the degradation or turnover of active vitamin D drugs. For example, some inactive vitamin D drugs competitively inhibit the 1,25-dihydroxyvitamin D₃ 24-hydroxylase (also known as vitamin D 24-hydroxylase, CYP24, and CYP24A1). Examples of inactive vitamin D drugs that competitively inhibit the vitamin D 24-hydroxylase include vitamin D drugs that lack a hydroxyl group at the C1 position and that, optionally, are hydroxylated at the C-25 and/or the C-24 positions, such as 25-hydroxyvitamin D or 24,25-dihydroxyvitamin D. The numbering of carbons for vitamin D and its analogs discussed herein or otherwise known in the art is as shown in Formula I.

Another subset of inactive vitamin D drugs that increase the blood, tissue, or cellular level of active vitamin D drugs include those that serve as a substrate for the production of 1,25-dihydroxyvitamin D compounds or an active analogs thereof. Examples of such vitamin D drugs include 25-hydroxylated vitamin D compounds such as 25-hydroxyvitamin D₂, 25-hydroxyvitamin D₃, 25-hydroxyvitamin D₄, and 25-hydroxyvitamin D₅. Many types of cells in the body express 25-hydroxyvitamin-D-1 alpha-hydroxylase. This enzyme is responsible for generating 1,25-dihydroxyvitamin D compounds from 25-hydroxyvitamin D in an autocrine manner. If tissues are not provided with adequate levels of 25-hydroxyvitamin D, the 25-hydroxyvitamin-D-1 alpha-hydroxylase enzyme can be substrate-deprived, and those tissues do not produce sufficient 1,25-dihydroxyvitamin D to prevent hyperproliferative, autoimmune, or infectious diseases. Conversely, prodrugs comprising 25-hydroxyvitamin D compounds as vitamin D-drug moieties can provide very high levels of the 25-hydroxyvitamin D to localized areas, such as the lower intestine, to ensure adequate substrate for local (autocrine) production of 1,25-dihydroxyvitamin D.

"Cleaved" or "free" vitamin D-drug moiety refers to vitamin D drugs derived from cleavage of a vitamin D prodrug, wherein the vitamin D-drug moiety is cleaved from the pro moiety.

"Cells that express (or contain) the vitamin D receptor" are those cells that have been shown to contain the vitamin D receptor, cells that are subsequently shown to contain the receptor (using immunohistochemical or other techniques), cell types (such as breast cancer cells) that have demonstrated a clinical improvement in response to treatment with calcitriol or its analogs or other vitamin D drugs, and cells for which there is epidemiologic data demonstrating an association between low vitamin D levels and higher disease incidence (such as adenocarcinomas of the prostate, breast, and colorectum). The presence of vitamin D receptors can be determined by any means known in the art, such as any of the techniques disclosed in Pike, Ann. Rev. Nutr. 1991 11:189-216.

The terms "target site," "target tissue" or "target cell" refer to a desired site, tissue, or cell in the body for treatment with or placement of a vitamin D drug. "Target site" encompasses both target tissues and target cells as well as any other generalized site in the body.

The term "treat" or "treatment" refers to repair, alleviation, or amelioration of a disease or condition in a target site. Examples include inhibition of abnormal growth, such as hyperproliferation of cells, promotion of cell differentiation, and modulation of immune cell function.

The term "therapeutic agent" refers to a material which has or exhibits healing powers when administered to or is delivered to the target site.

"Hypercalcemia" refers to a calcium plasma concentration greater than normal in the laboratory where the concentration is measured, for example greater than about 10.5 mg/dL in humans (although this and all other normal values can vary depending on the techniques used to measure the concentration). Examples of plasma calcium concentrations constituting hypercalcemia in other organisms are well known in the art. Hypercalcemia can be broken into grades 0-4, as set forth in the National Cancer Institute Common Toxicity Criteria summarized in Table 1.

"Symptomatic hypercalcemia" refers to laboratory-demonstrated hypercalcemia associated with one of more of the signs or symptoms of hypercalcemia. Early manifestations of hypercalcemia include weakness, headache, somnolence, nausea, vomiting, dry mouth, constipation, muscle pain, bone pain, or metallic taste. Late manifestations include polydypsia, polyuria, weight loss, pancreatitis, photophobia, pruritis, renal dysfunction, aminotransferase elevation, hypertension, cardiac arrhythmias, psychosis, stupor, or coma. Ectopic calcification has been reported when the calcium-phosphate product (multiplying the concentrations of calcium and phosphate in mg/dl) exceeds 70. Symptomatic hypercalcemia can be broken into grades 0-4, as set forth in the National Cancer Institute Common Toxicity Criteria summarized in Table 1. "Severe symptomatic hypercalcemia" refers to grade 3 or grade 4 hypercalcemia.

**Table 1. National Cancer Institute Common Toxicity Criteria**

| **Toxicity Grade** | **0** | **1** | **2** | **3** | **4** |
|---|---|---|---|---|---|
| **Blood/Bone Marrow** | | | | | |
| WBC | >4.0K | 3.0-3.9K | 2.0-2.9K | 1.0-1.9K | <1K |
| Platelets | WNL | 75.0K-WNL | 50-74.9K | 25.0-49.9K | <25K |
| Hemoglobin | WNL | 10.0 g-WNL | 8.0-10.0 | 6.5-7.9 g | <6.5 g |
| Neutrophils | >2.0K | 1.5-1.9K | 1.0-1.4K | 0.5-0.9K | <0.5K |
| Lymphocytes | >2.0K | 1.5-1.9K | 1.0-1.4K | 0.5-0.9K | <0.5K |
| Hemorrhage | None | Mild, No | Gross, 1-2 U | Gross, 3-4 U | Massive, >4U |
| Clinical | | Transfusion s | PRBC | PRBC | PRBC |
| Infection | None | Mild | Moderate | Severe | Life-Threatening |

| **Gastrointestinal** | | | | | |
|---|---|---|---|---|---|
| Nausea | None | Able to Eat | Intake Decreased | No Significant Intake | |
| Vomiting | None | 1x/24 hours | 2-5x/24 hours | 6-10x/24 hrs | >10x/24 hrs |
| Diarrhea | None | Increase of 2-3x/24 hours | Increase of 4-6x/24 hours | Increase of 7-9x24 hours | Increase of >10x/24 hrs |
| Stomatitis | None | Painless Ulcers | Painful Ulcers, Can Eat | Painful Ulcers, Cannot Eat | Requires IV Nutrition |

| **Hepatic** | | | | | |
|---|---|---|---|---|---|
| Bilirubin | WNL | | <1.5x WNL | 1.5-3.0x WNL | >3x WNL |
| SGOT/SGPT | WNL | <2.5x WNL | 2.6-5.0x WNL | 5.1-20 WNL | >20x WNL |
| Alk Phos | WNL | <2.5x WNL | 2.6-5.0x WNL | 5.1-20 WNL | >20x WNL |
| Liver/Clinical | No Change | | | Precoma | Hepatic Coma |

| **Kidney/Bladder** | | | | | |
|---|---|---|---|---|---|
| Creatinine | WNL | <1.5x WNL | 1.5-3.0x WNL | 3.1-6.0x WNL | >6.0x WNL |
| Proteinuria | No Change | 1 + <0.3 gm % | 2-3 + 0.3-1.0 gm % | 4 + >1.0 gm % | Nephrotic Syndrome |
| Hematuria | Negative | Microscopic | Gross | With Clots | Transfusion |
| Alopecia | No Loss | Mild | Total | | |

| **Cardiovascular** | | | | | |
|---|---|---|---|---|---|
| Dysrhythmia | None | Asymptoma tic No Therapy | Persistent No Therapy | Requires Therapy | Hypotensio n, V-tach/V-fib |
| Cardiac | None | Decline of EF by <20% | Decline of EF by >20% | Mild CHF, Rx Responsive | Refractory CHF |

| **Toxicity Grade** | 0 | 1 | 2 | 3 | 4 |
|---|---|---|---|---|---|
| Ischemia | None | Nonspecific ST-T Wave changes | Asymptomatic Ischemic changes | Angina, No Infarction | Acute MI |
| Pericardial | None | Asymptoma tic Effusion | Pericarditis, rub, EKG changes | Symptomatic Effusion | Tamponade |
| Hypertension | None | Transient, >20 mm Hg | Persistent, >20 mm, No Rx | Requires Therapy | Hypertensive Crisis |
| Hypotension | None | Transient, No Therapy | Fluid Replacement | Hospitalized <48 Hours | Hospitalized >48 Hours |
| Pulmonary | No Change | Asymptomatic Abnormal PFT | Dyspnea on Exertion | Dyspnea, no exertion | Dyspnea at Rest |

| **Neurologic** | | | | | |
|---|---|---|---|---|---|
| Neuro-sensory | No Change | Mild Paresthesia | Moderate Sensory Loss | Severe Loss, Symptomatic | |
| Neuro-motor | No Change | Subjective Weakness | Mold Objective Weakness | Impairment of Function | Paralysis |
| Coritcal | None | Mild Somnolense, Agitation | Moderate Somnolence, Agitation | Contusion or Hallucinatio n | Coma or Seizures |
| Cerebellar | None | Slight Change Coordination | Speech Slur, Tremor, Nystagmus | Ataxia | Cerebellar Necrosis |
| Mood | No Change | Mild Anxiety or Depression | Moderate | Severe | Suicidal |
| Headache | None | Mild | Transient, Moderate-Severe | Unrelenting, Severe | |
| Constipation | None | Mild | Moderate | Severe | Ileus >96 Hrs |
| Hearing | No Change | Asymptomatic Audiometry changes | Tinnitus | Correctable Loss | Deaf, not Correctable |
| Vision | No Change | | | Symptomatic Subtotal Loss | Blindness |

| **Toxicity Grade** | **0** | **1** | **2** | **3** | **4** |
|---|---|---|---|---|---|
| Skin | No Change | Macular/ Papular Rash, Asymptomati c | Rash with Pruritus | Generalized Eruption | Exfoliative or Ulcerative Rash |
| Allergy | None | Transient Rash, Temp <38 °C | Urticaria, Mild Bronchospasm, T>38° C | Serum Sickness, Bronchospas m | Anaphylaxis |
| Fever | None | 37.1-38°C | 38.1-40°C | 40°C, <24 Hrs | >40°, >24 Hrs |
| Local | None | Pain | Inflammation Phlebitis | Ulceration | Plastic Surgery Rx |
| Weight Change | <5% | 10-19.9% | >20% | | |

| **Metabolic** | | | | | |
|---|---|---|---|---|---|
| HyperGlycemia | <116 | 116-160 | 161-250 | 251-500 | >500, Ketoacidosi s |
| Hypoglycemia | >64 | 55-64 | 40-54 | 30-39 | <30 |
| Amylase | WNL | <1.5x WNL | 1.5-2.0x WNL | 2.1-5.0x WNL | >5.1xWNL |
| HyperCalcemia | <10.6 | 10.6-11.5 | 11.6-12.5 | 12.6-13.5 | >13.5 |
| Hypocalcemia | <8.4 | 8.4-7.8 | 7.7-7.0 | 6.9-6.1 | <6.0 |
| Hypo-Magnesemia | >1.4 | 1.4-1.2 | 1.1-0.9 | 0.8-0.6 | <0.5 |

| **Coagulation** | | | | | |
|---|---|---|---|---|---|
| Fibrinogen | WNL | .75-1x WNL | .5-7.4x WNL | .25-.49x WNL | 24x WNL |
| PT | WNL | 1-1.25x WNL | 1.26-1.5x WNL | 1.51-2.0x WNL | >2.0x WNL |
| PTT | WNL | 1-1.25x WNL | 1.2-1.5x WNL | 1.51-2.0x WNL | >2.0x WNL |

A "vitamin D-sensitive disease" refers to any disease or condition known or discovered that responds to active forms of vitamin D, such as 1,25-dihydroxyvitamin D₂, 1,25-dihydroxyvitamin D₃, 1,25-dihydroxyvitamin D₄, or 1,25-dihydroxyvitamin D₅.

A "tumour" is a neoplasm, and includes both solid and non-solid tumours (such as hematologic malignancies). A "hyperproliferative disease" is a disorder characterized by abnormal proliferation of cells, and generically includes skin disorders as well as benign and malignant tumours of all organ systems. "Differentiation" refers to the process by which cells become more specialized to perform biological functions, and differentiation is a property that is totally or partially lost by cells that have undergone malignant transformation.

A "therapeutically effective dose" is a dose which in susceptible subjects is sufficient to prevent advancement of a disease or to cause regression of the disease, or which is capable of relieving symptoms caused by the disease, such as fever, pain, decreased appetite, or cachexia associated with disease.

A "therapeutic effect" is the prevention of advancement of a disease, regression of a disease, or relief of symptoms caused by a disease.

"Potentiates" used with reference to activity of a first vitamin D prodrug with respect to a second vitamin D prodrug means that the first vitamin D prodrug is ineffective on its own in eliciting a therapeutic effect at a target tissue but increases the effectiveness of the second vitamin D prodrug when administered therewith.

The "calcemic index" of a drug is a measure of the relative ability of a drug to generate a calcemic response, for example as measured and reported in Bouillon et al., Endocrine Reviews. 1995 16:200-257. A calcemic index of 1 corresponds to the relative calcemic activity of calcitriol. A calcemic index of about 0.01 corresponds to the calcemic activity of calcipotriol. A calcemic index of 0.5 would correspond to a drug having approximately half the calcemic activity of calcitriol. The calcemic index of a drug can vary depending on the assay conducted, e.g. whether measuring stimulation of intestinal calcium absorption (ICA) or bone calcium mobilizing activity (BCM), as reported in Hurwitz et al., J. Nutr.1967 91:319-323 and Yamada et al., Molecular, Cellular and Clinical Endocrinology (Berlin), 1988 767-774. Hence relative calcemic activity is best expressed in relation to the calcemic activity of calcitriol, which is one of the best characterized vitamin D drugs.

The vitamin D prodrugs that may be used in the present invention include compounds having a vitamin D drug as a vitamin D-drug moiety and further having a glycone or sulfate moiety as a pro moiety. These include the prodrugs defined according to Formula (I): 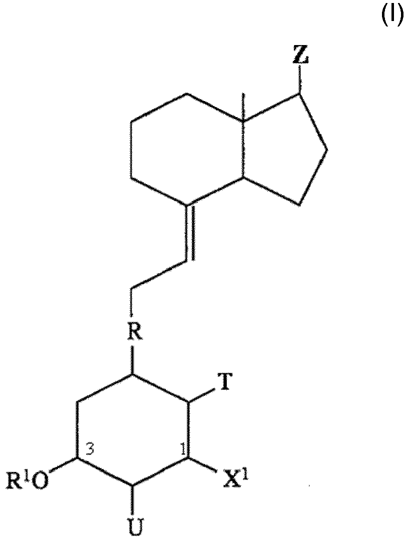 wherein T is hydrogen or a =CH₂ group;
- X¹: is selected from the group consisting of -OH, and -OR¹;
- U: is hydrogen, C₁-C₆ alkenyl, C₁-C₆ alkyl, -OH, or -O-(C₂-C₄ alkyl)-OH;
- R: is a double bond or an epoxy group;
- R¹: is hydrogen, -SO₃, or a straight- or branched-chain glycone moiety comprising 1-20 glycone units, or R¹ is an orthoester glycoside moiety of Formula (II): 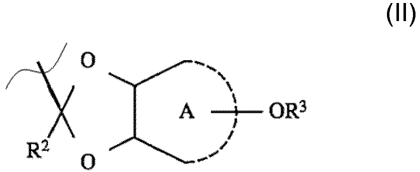 wherein A represents a glycofuranosyl or glycopyranosyl ring;
- R²: is hydrogen, lower alkyl, aralkyl, or aryl, with the proviso that aryl is phenyl or phenyl substituted by chloro, fluoro, bromo, iodo, lower C₁-C₄ alkyl, C₁-C₄ alkoxy; or naphthyl; and
- R³: is hydrogen, -SO₃, or a straight- or branched-chain glycone moiety comprising 1-20 glycone units;
- Z: is a hydrogen or a saturated or unsaturated, substituted or unsubstituted, straight-chain or branched C₁-C₁₈ hydrocarbon group, preferably having a formula as represented by Formula (III): 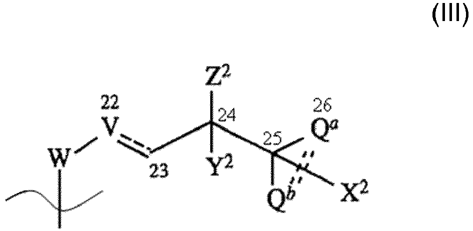 wherein the bond between C-22 and C-23 is a single or double bond;
- Y²: is hydrogen, fluorine, C₁-C₆ alkyl, -OH, or -OR¹;
- Z²: is hydrogen, fluorine, C₁-C₆ alkyl, -OH, or -OR¹;
- Q*^{a}*: is -CF₃ or -CH₂X²;
- Q*^{b}*: is -CF₃ or -CH₃;
- X²: is hydrogen, -OH, or -OR¹;
- W: is -CH-CH₃ or -O-;
- V: is CH₂ or -O-, wherein W and V are not both -O-; and
"= = =" is either a single bond between Q^{a} and Q^{b} or a hydrogen atom on both Q^{a} and Q^{b}, wherein when "= = =" is a single bond, X² is H;
wherein at least one of the R¹ comprises at least one glycone moiety or at least one -SO₃ moiety.

In Formula I, "alkyl" indicates linear and branched chains. The vitamin D-drug moiety of Formula I comprises any portion that is not explicitly defined as a glycone moiety, an orthoester glycoside moiety, or a -SO₃ group.

When the compounds of Formula I have a double bond between C-22 (V in Formula I) and C-23, and a methyl group at C-24 (*i.e*., at Z² or Y²), they are derivatives of vitamin D₂. When the compounds of Formula (I) have a single bond between C-22 and C-23, and no C-24 alkyl (*i.e*., Z² or Y² is not C₁-C₆ alkyl), they are derivatives of vitamin D₃. When the compounds of Formula (I) have a single bond between C-22 and C-23 and a C₁-C₆ alkyl appended to C-24 (*i.e*., at Z² or Y² is C₁-C₆ alkyl), they are derivatives of vitamin D₄ or vitamin D₅.

Preferred vitamin D-drug moieties are those derived from vitamins D₂, D₃, D₄, or D₅, including but not limited to those derived from 1α-hydroxyvitamins D₂, D₃, D₄, or D₅; 25-hydroxyvitamins D₂, D₃, D₄, or D₅; 1α,24-dihydroxyvitamins D₂, D₃, D₄, or D₅; 1α,25-dihydroxyvitamins D₂, D₃, D₄, or D₅; 24,25-dihydroxyvitamins D₂, D₃, D₄, or D₅; 25,26-hydroxyvitamins D₂, D₃, D₄, or D₅; 1α,24,25-trihydroxyvitamins D₂, D₃, D₄, or D₅; and 1α,25,26-trihydroxyvitamins D₂, D₃, D₄, or D₅. Among the most preferred are the vitamin D-drug moieties derived from 1α-hydroxyvitamins D₂ or D₃; 1α,25-dihydroxyvitamins D₂ or D₃; 25-hydroxyvitamin D₂ or D₃; 24,25-dihydroxyvitamin D₂ or D₃; 1α,24-dihydroxyvitamin D₃; 5,6-epoxy derivatives of vitamin D and its metabolites; 2-β-(3-hydroxypropoxy)-1α,25-dihydroxyvitamin D₃; and the side chain fluoro derivatives of 1α,25-(OH)₂ vitamin D and 1α-(OH) vitamin D. Also preferred are 20- and 22-oxa vitamin D derivatives including 20-oxa-1α(OH)D, 20-oxa-1α,25(OH)₂ D₃, 22-oxa-1α(OH)D₃ and 22-oxa-1α,25(OH)D₃ as well as pseudo-1α-hydroxyvitamin D derivatives such as dihydrotachysterol and 5,6-trans vitamin D₃ and their 25-hydroxy derivatives. Also preferred is calcipotriol having the formula: 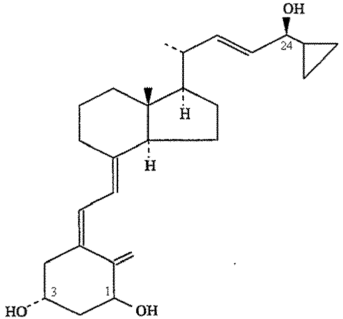 (see Krayballe, K., Arch. Dertnatol. 1989 125:1647), wherein a pro moiety can be linked via a hydroxy group at positions 1, 3, and/or 24. Also preferred are vitamin D analogs 1,25-dihydroxy-16-ene-23-yne-26 and 27-hexafluorocholecalciferol. Additional vitamin D-drug moieties, and methods for producing them, include those described in U.S. Pat. No. 6,929,797. Other preferred vitamin D-drug moieties are described elsewhere in this application.

Other suitable vitamin D-drug moieties include: 1α,25-(OH)₂-26-27-d_{g}-D₃; 1α,25-(OH)₂-25-eme-D₃; 1α,25-(OH)₂-D₃; 1α,25-(OH)₂-26,27-F₆-22-ene-D₃; 1α,25-(OH)₂-26,27-F₆-D₃; 1α,25S-(OH)₂-26-F₆-D₃; 1α,25-(OH)₂-24-F₆-D₃; 1α,25-26-(OH)₂-22-ene-D₃; 1α,25R,26-(OH)₂-22-ene-D₃; 1α,25-(OH)₂-D₃; 1α,25-(OH)₂-24-epi-D₃; 1α,25-(OH)₂-23-ync-D₃; 1α,25-(OH)₂-24R-F-D₃; 1α,25S,26-(OH)₂-D₃; 1α,23S,25-(OH)₂-D₃; 1α,23R,25-(OH)₂-D₃; 1α,24R-(OH)₂-25F-D₃; 1α,25-(OH)₂-26,27-F₆-23-yne-D₃; 1α,25R-(OH)₂-26-F₃-D₃; 1α,25,28-(OH)₂-D₃; 1α,25-(OH)₂-16-Ene-23-yne-D₃; 1α,24R,25-(OH)₂-D₃; 1α,25-(OH)₂-26,27-F₆-23-ene-D₃; 25-(OH)-23-Yne-D₃; 25-(OH)-26,27-F₆-23-yne-D₃; 1α,25R-(OH)₂-22-Ene-26 F₆-D₃; 1α,25S-(OH)₂-22-Ene-26-F₆-D₃; 1α,25R-(OH)₂-D₃-26,26,26-d₃; 1α,25S-(OH)₂-D₃-26,26,26-d₃; 1α,25R-(OH)₂-22-Ene-D₃-26,26,26-d₃; 1α,25S-(OH)₂-22-Ene-D₃-26,26,26-d₃; 1α,25-(OH)₂-D₃-26,26,26-27,27,27-d₃; 1α,25-(OH)₂-24-Epi-D₃-26,26,26,27,27,27-d₃; 1α,25-(OH)₂-D₃-23,23,24,24,26,26,26,27,27,27-d₃; 1α,25-(OH)₂-22-Ene-D₃-26,26,26,27,27,27-d₃; (11)-Dehydro-3-deoxy-1,25--(OH)₂-D₃; 2-Nor-1,3-seco-1,25-(OH)₂-D₃; 2,4-Dinor-1,3-seco-1,25-(OH)₂-D₃; 1,1-Dimethyl-2,4-dinor-1,3-seco-1,25-(OH)₂-3-Deoxy-2-oxα-25-(OH)₂-D₃; 24R,25-(OH)₂-D₃; 25-(OH)-16 Ene-23-yne-D₃; 1-F-25-(OH)-16-ene-23-yne-D₃; 1α,25-(OH)₂-16-Ene-23-yne-D₃-26,26,26,27,27,27-d₃; 1-F-25-(OH)-16-ene-23-yne-D₃-26,26,26,27,27,27-d₃; A-Homo-2-deoxy-3,3-dimethyl-2,4-dioxa-25-(OH)₂-D₃; 24-Nor-1α,25-(OH)₂-D₃; 25-Oxo-25-phospha-D₃; (11)-Dehydro-11-(4-hydroxymethylphenyl)-1,25-(OH)₂-D₃; (23S,25S)-1α,25-(OH)₂-D₃-26,23-lactone; 1α,11β,25-(OH)₂-D₃; (11) Dehydro-11(3-hydroxypropyn-1-yl)-1,25-(OH)₂-D₃; (11)-Dehydro-11(3-acctoxyropyn-1-yl)-1,25-(OH)₂-D₃; (11)-Dehydro-11(4-acetoxymethylphenyl)-1,25-(OH)₂-D₃; Vitamin-D₃; 25-(OH)₂-D₃; 1α-(OH)₂-D₃; (23R,25S)-1α-(OH)₂-D₃-26,23-lactone; (23R,25R)-1α,25-(OH)₂-D₃-26,23-lactone; (23S,25R)-1α,25-(OH)₂-D₃-26,23-lactone [Natural Form]; 1α,24S-(OH)₂-22-Ene-26,27-dehydro-D₃; (11)-Dehydro-1α-25-(OH)₂-D₃; 1α-11α,25-(OH)₂-D₃; 11β-Methoxy-1α,25-(OH)₂-D₃; 11α-Methoxy-1α,25-(OH)₂-D₃; 25-(OH)₂-23-Oxa-D₃; 1α,24S,25-(OH)₂-D₃; 3-Deoxy-1α,25-(OH)₂-D₃; 1α,24R-(OH)₂-D₃; 1α,24S-(OH)₂-D₃; 1α,25-(OH)₂-24-Oxo-D₃; 1α,23,25-(OH)₂-24-Oxo-D₃; 1α-(OH)-25-Oxo-25-phospha-D₃; 25-Oxo-26,27-dimethyl-25-phospha-26,27-dioxa-D₃; 1α-(OH)-25-Oxo-26,27-dimethyl-25-phospha-26,27-dioxa-D₃; 22-(Meta-hydroxyphenyl)-1α,25-(OH)₂-D₃; 22-(Para-hydroxphenyl)-1α,25-(OH)₂-D₃; 1α,25-(OH)₂-5,6-trans-D₃; 25R,26-(OH)₂-D₃; 25S,26-(OH)₂-D₃; 1α,25S,26-(OH)₂-D₃; 1α,25R,26-(OH)₂-D₃; (23R,25S)-25-(OH)₂-D₃-26,23-lactone; (23S,25R)-25-(OH)-D₃-26,23-lactone; 6-Flouro-D₃; 1α,25-(OH)₂-16-Ene-23-yne-26,27-F₆-D₃; 25-(OH)-16-Ene-23-yne-26,27-F₆-D₃; 1α,F-25-(OH)-16-Ene-23-yne-26,27-F₆-D₃; 1α,25-(OH)₂-24α-Homo-D₃; 1α,25-(OH)₂-24α-Dihomo-D₃; 22-(m-methylphenyl)-23,24,25,26,27-pentanor-1α,-(OH)₂-D₃; 22-Oxa-1α,25-(OH)₂-D₃; 22 (m-(dimethylhydroxymethyl)phenyl) 23,24,25,26,27-pentanor-1α,-(OH)₂-D₃; 1α,25-(OH)₂-22-Ene-D₃; 25-(OH)-23-Ene-D₃; 1α,25-(OH)₂-16,23(E)-diene-D₃; 14-Epi-1α,25-(OH)₂-D₃; 14-Epi-1α,25-(OH)₂-pre-D₃; 3-Deoxy-3-thia-1α,25-(OH)₂-D₃; 3-Deoxy-3-thia-1β,25-(OH)₂-D₃; 1α,25-(OH)₂-pre-D₃-9,14,19,19-D₃; 1α,25-(OH)₂-D₃-9,9,14,19,19-D₃; 1β,25-(OH)₂-epi-D₃; 1α,-25-(OH)₂-6,7-Dehydro-pre-D₃; 1α,25-(OH)₂-3-epi-D₃; 1β,25-(OH)₂-6,7-Dehydro-3-epi-pre-D₃; 1β,25-(OH)₂-D₃; 1α,25-(OH)₂-16-Ene-D₃; 25-(OH)-16-Ene-D₃; 25-(OH)-16,23-Diene-D₃; 1α,2,25-(OH)₂-D₃; (22S)-1α,25-(OH)₂-22,23-Diene-D₃; (22R)-1α,25-(OH)₂-22,23-Diene-D₃; 1α,18,25-(OH)₂-D₃; 1α,18-(OH)₂-D₃; 18-Acetoxy-1α,25-(OH)₂-D₃; 18-Aceotxy-1α,(OH)₂-D₃; 23-(m-Dimethylhydroxymethyl)-22 - yne-24,25,26,27-tetraanor-1α,-(OH)₂-D₃; 24α,26α,27α-Trihomo-22,24-diene-1α,-(OH)₂-D₃; 20-Epi-22-oxa-24α,26α,27α-trihomo-1α,-25-(OH)₂-D₃; 20-Epi-1α,-(OH)₂-D₃; 20-Epi-24α,26α,27α-trihomo-1α,-25-(OH)₂-D₃; 18-oxo-1α,-25-(OH)₂-D₃; 3-Deoxy-3-thia-1α,-25-(OH)₂-D3β-oxide; 5,6-trans-3-Deoxy-3-thia-1α,-25-(OH)₂-D₃β-oxide; 24α-Homo-22,24(14α)-diene-1α,25-(OH)₂D₃; 24α-Dihomo-1α,22R,25-(OH)₂D₃; 8,(14α)-homo-1α,25-(OH)₂D₃; 23-oxa-1α,25-(OH)₂D₃; 1α-(hydroxymethyl)-25-(OH)₂D₃; 1β-(hydroxymethyl)-3α,25-(OH)₂D₃; 2β-(3-hydroxypropoxy)-1α,25-(OH)₂D₃; 1α,25-(OH)₂-24(S)-5,6-D₃; 1α,25-(OH)₂-24(R)-5,6-D₃; 11α-phenyl-1α,25-(OH)₂D₃;11β-phenyl-1α,25-(OH)₂D₃; 11α-dimethylaminophenyl-1α,25-(OH)₂D₃; 11α-methyl-1α,25-(OH)₂D₃; 11β-methyl-1α,25-(OH)₂D₃; 11α-hydroxymethyl-1α,25-(OH)₂D₃; 11α-fluoromethyl-1α,25-(OH)₂D₃; 11α-chloromethyl-1α,25-(OH)₂D₃; 11α-ethyl-1α,25-(OH)₂D₃; 11α-(2-hydroxyethyl)-1α,25-(OH)₂D₃; 11β-(2-hydroxyethyl)-1α,25-(OH)₂D₃; 11α-vinyl-1α,25-(OH)₂D₃; 11α-ethynl-1α,25-(OH)₂D₃; 11α-[11(R)-oxacyclopropyl]-1α,25-(OH)₂D₃; 11α-[11(S)-oxacyclopropyl]-1α,25-(OH)₂D₃; 1α,25-(OH)₂-13-vinyl-18-nor-D₃; 25-(OH)-16,23(Z)-diene-D₃; 1α,25-(OH)₂-16,23(Z)-diene-D₃; 1α,25-(OH)₂-18-methyl-D₃; 1α,25-(OH)₂-19-nor-pre-D₃; 1α,25-(OH)₂₋19-nor-D₃; 15,26-epoxy-23-yne-19-nor-1α-(OH)₂-D₃; 20-epi-24-homo-1α,25-(OH)₂-D₃; 20-epi-22-oxa-1α,25-(OH)₂-D₃; 20-epi-22-oxa-24-homo-1α,25-(OH)₂-D₃; 20-epi-22-oxa-24-dihomo-1α,25-(OH)₂-D₃; 20-epi-22-oxa-24-dihomo-26,27-dihomo-1α,25-(OH)₂-D₃; 20-epi-23-oxa-24α,24b-dihomo-1α,25-(OH)₂-D₃; 25,26-epoxy-23-yne-20-epi-1α-(OH)₂-D₃; 1α-(OH)-20-oxa-21-nor-D₃; 1α-25-(OH)₂-20-oxa-21-nor-D₃; 22-oxa-1α-(OH)-D₃; 1α,24(S)-(OH)₂-22-oxa-D₃; 1α,24(S)-(OH)₂-22-oxa-26,27-dimethyl-D₃; 1α,25-(OH)₂-22-oxa-26,27-dimethyl-D₃; 22-(OH)-D₃; 1α-(OH)-22-oxa-D₃; 23,24,25,26,27-pentanor-1,22-(OH)₂-D₃; 1α-(OH)-22-E-ene-D₃; 1α-(OH)-22-Z-ene-D₃; 1α,25(OH)₂-22-ene-24-homo-D₃; 1α,25-(OH)₂-22-ene-24,24-dihomo-D₃; 22-dehydro-24,24,24-trihome-1α,25-(OH)₂-D₃; 26-homo-22-dehydro-1α,25(R)-(OH)₂-D₃; 1α-(OH)-22-ene-24-oxo-26,27-dehydro-D₃; (24S,25S)-25,26-epoxy-22-ene-1α,24-(OH)₂-D₃; (24S,25R)-25,26-epoxy-22-ene-1α,24-(OH)₂-D₃; (22E,24R-1α,24-(OH)₂-22-dehydro-D₃; (22E,24s-1α,24-(OH)₂-22-dehydro-D₃; 24,25-epoxy-22-yne-1α-OH)-D₃; 23,24-dinor-1,25-(OH)₂-D₃; 23-oxa-24α,24b-dihomo-1α,25-(OH)₂-D₃; 23-thia-1α,25-(OH)₂-D₃; 23-axa-1α,25-(OH)₂-D₃; 24,25-epoxy-26,27-dinor-23,23-dimethyl-1α-(OH)-D₃; 1α,23-(OH)₂-25,26-dehydro-D₃; 23-keto-25-(OH)-D₃; 23(S)-OH-26,27-F₆-1α,25-(OH)₂-D₃; 24,25,26,27-tetranor-1,23-(OH)₂-D₃; 23(S),25(R)-1α,25-(OH)₂-D₃-26,23-lactol; 1α,25-(OH)₂-16,23 (Z)-diene-D₃; 25,26-epoxy-23-yne-1α-(OH)-D₃; 1α,25-(OH)₂-24,26,27-trihomo-D₃; 22-oxa-24,26,27-trihomo-1α,25-(OH)₂-D₃; 24,24-difluoro-24-homo-1α,25-(OH)₂-D₃; 24R-(OH)-25-F-D₃; 26,27-F6-1α,24-(OH)₂-D₃; (24S,25S)-25,26-epoxy-1α,24-(OH)₂-D₃; (24R,25R)-25,26-epoxy-1α,24-(OH)₂-D₃; (24S,25R)-25,26-epoxy-1α,24-(OH)₂-D₃; (24R,25S)-25,26-epoxy-1α,24-(OH)₂-D₃; (24R,25S)-25,26-epoxy-27-nor-1α,24-(OH)₂-D₃; (24S,25R)-25,26-epoxy-27-nor-1α,24-(OH)₂-D₃; 24,25-epoxy-1α-(OH)₂-D₃; 24-ene-D₃; 1α-(OH)-24-ene-D₃; 24,24-difluoro-1α,25(OH)₂-26,27-dimethyl-D₃; 22,23-dihydro-24-epi-1α,25-(OH)₂-D₃; 25,26,27-trinor-1α,25-(OH)₂-D₃; 25-aza-D₃; 25,26-epoxy-1α-(OH)-D₃; 1α-(OH)-25-hydroxymethyl-D₃; 1α-(OH)-25-F-D₃; 1α,25-FZ-D₃; 1α,25-(OH)₂-26-homo-D₃; 26,27-dimethyl-1α,25-(OH)₂-D₃; 26,27-diethyl-1α,25-(OH)₂-D₃; 26,27-dipropyl-1α,25-(OH)₂-D₃; 1α,23(S),25(R),26-(OH)₂-D₃; 1α-(OH)-26,27-F₆-D₃; 25-(OH)-26,27-F₆-D₃; 26,27-dinor-1α-25-(OH)-D₃; 1α-(OH)-24-axo-26,27-dehydro-D₃; 23-oxa-26,27-dimethyl-1α-(OH)-D₃; 20-ene-23-oxa-26,27-dimethyl-1α-25-(OH)₂D₃; 20,21-methano-23-oxa-26,27-dimethyl-1α-25-(OH)₂D₃; 20-methyl-23-oxa-26,27-dimethyl-1α-25-(OH)₂D₃; 22-ene-26-methyl-1α-25S(OH)₂D₃; 22-ene-26,27-dimethyl-1α-25-(OH)₂D₃; 22-ene-24-homo-26,27-dimethyl-1α-25-(OH)₂D₃; 20-epi-22-ene-24-homo-26,27-dimethyl-1α-25-(OH)₂D₃; 22-yne-26,27-dimethyl-1α-25-(OH)₂D₃; 22-yne,24-homo-26,27-dimethyl-1α-25-(OH)₂D₃; 22-yne-24-dihomo-26,27-dimethyl-1α-25-(OH)₂D₃; 20-epi-22-yne-26,27-dimethyl-1α,25-(OH)₂D₃; 20-epi-22-yne-24-homo-26,27-dimethyl-1α-25-(OH)₂D₃; 20-epi-22-yne-24-dihomo-26,27-dimethyl-1α-25-(OH)₂D₃; 20-epi-22-yne-24-trihomo-26,27-dimethyl-1α-25-(OH)₂D₃; 17(20)E-ene-22-yne-24-homo-26,27-dimethyl-1α-25-(OH)₂D₃; 17(20)Z-ene-22-yne-26,27-dimethyl-1α-25-(OH)₂D₃; 17(20)Z-ene-22-yne-24-homo-26,27-dimethyl-1α-25-(OH)₂D₃; 17(20)Z-ene-22-yne-24-dihomo-26,27-dimethyl-1α-25-(OH)₂D₃; 20-ene-22-yne-26,27-dimethyl-1α-25-(OH)₂D₃; 20-ene-22-yne-24-homo-26,27-dimethyl-1α-25-(OH)₂D₃; 26,27-dimethyl-1α-20,25-(OH)₂D₃; 24-homo-26,27-dimethyl-1α-20,25-(OH)₂D₃; 20-methoxy-26,27-dimethyl-1α-25-(OH)₂D₃; 20-methoxy-24-homo-26,27-dimethyl-1α-25-(OH)₂D₃; 20-ethoxy-26,27-dimethyl-1α-25-(OH)₂D₃; 20-ethoxy-24-homo-26,27-dimethyl-1α-25-(OH)₂D₃; 26,27-dimethyl-1α-22S,25-(OH)₂D₃; 24-homo-26,27-dimethyl-1α-22S,25-(OH)₂D₃; 24-dihomo-26,27-dimethyl-1α-22S,25(OH)₂D₃; 23-yne-24-dihomo-26,27-dimethyl-1α-22S,25-(OH)₂D₃; 23-yne-24-trihomo-26,27-dimethyl-1α-22S,25-(OH)₂D₃; 23-yne-26,27-dimethyl-1α-22S,25-(OH)₂D₃; 23-yne-24-homo-26,27-dimethyl-1α-22S,25-(OH)₂D₃; 23-yne-24-dihomo-26,27-dimethyl-1α-22S,25-(OH)₂D₃; 23-yne-24-trihomo-26,27-dimethyl-1α-22S,25-(OH)₂D₃; 22R-methoxy-23-yne-26,27-dimethyl-1α,25-(OH)₂D₃; 22R-methoxy-23-yne-24-homo-26,27-dimethyl-1α,25-(OH)₂D₃; 23-oxa-26,27-diethyl-1α,25-(OH)₂D₃; 20-ene-23-oxa-26,27-diethyl-1α,25-(OH)₂D₃; 20,21-methane-23-oxa-26,27-diethyl-1α,25-(OH)₂D₃; 20-epi-22-oxa-24-dihomo-26,27-diethyl-1α,25-(OH)₂D₃; 26,27-diethyl-1α,20,25-(OH)₂D₃; 20-methoxy-26,27-diethyl-1α,25-(OH)₂D₃; 22-ene-26,27-dehydro-1α,24R-(OH)₂D₃; 20-epi-22-ene-26,27-dehydro-1α,24S-(OH)₂D₃; 20-epi-22-ene-26,27-dehydro-1α,24R-(OH)₂D₃; 24-dihomo-26,27-diethyl-1α,25-(OH)₂D₃; 20-epi-22-oxa-24-homo-26,27-diethyl-1α,25-(OH)₂D₃; and 22-oxa-26,27-diethyl-1α,25-(OH)₂D₃.

The configuration of the oxygen linkage of a hydroxy group or pro moiety attached to the vitamin drug-moiety may be either α (out of the plane of the paper) or β (into the plane of the paper). In vitamin D-drug moieties comprising glycone groups (*i.e*., wherein R¹ or R³ is a glycone moiety) as pro moieties, the linkage can be α (out of the plane of the paper) or β (into the plane of the paper), but is preferably β. It is preferred if the configuration of the 3-hydroxy, sulfate, or glycosidoxy group at C-3 be β, and that, independently or simultaneously, the configuration of the hydroxy, sulfate, or glycosidoxy at C-1 be α. It is also preferred that the configuration around C-24 be R. When, at C-24, X=H and R²=-CH₃ the configuration at C-24 is preferably S.

The vitamin D prodrugs useful in the practice of the invention contain at least one, and up to five, pro moieties, which can be at any of positions 1, 3, 24, or 25 or, indirectly, at position 26 (see Formula I). In the case of multihydroxylated forms of the vitamin D drugs (*e.g*., 1,25-dihydroxyvitamin D₃ has three hydroxy groups: at positions 1, 3, and 25), the preferred vitamin D prodrugs are those wherein fewer than all of the multiple hydroxy groups include pro moieties and, most preferably, wherein only one of the multiple hydroxy groups comprises a pro moiety. For the purposes of this disclosure, it is understood that the pro moiety can be appended to any hydroxyl group existing in the cleaved (free) form of the vitamin D drug. For example, in 24,25-dihydroxyvitamin D₃, a pro moiety can be appended to the hydroxyl group at C-24, C-25, C-3, or any combination thereof.

In vitamin D-drug moieties comprising sulfate groups (*i.e*., wherein R¹ or R³ is -SO₃) as pro moieties, the linkage can be α (out of the plane of the paper) or β (into the plane of the paper), but is preferably β. The vitamin D-drug moieties can have sulfate groups at any of positions 1, 3, 24, or 25 or, indirectly, at position 26 in the carbon backbone (see Formula I).

By "glycone moiety" is meant glycopyranosyl or glycofuranosyl, as well as amino sugar derivatives thereof and other moieties discussed herein. The residues may be homopolymers or random, alternating, or block copolymers comprised of glycone units. The glycone units have free hydroxy groups, or hydroxy groups acylated with a group R⁴-(C=O)-, wherein R⁴ is hydrogen, lower C₁₋₆ alkyl, C₆₋₁₀ substituted or unsubstituted aryl, or C₇₋₁₆ aralkyl. Preferably, R⁴ is acetyl or propionyl; phenyl, nitrophenyl, halophenyl, lower alkyl substituted phenyl, lower alkoxy substituted phenyl, and the like; or benzyl, lower alkoxy substituted benzyl, and the like.

The glycopyranose or glycofuranose rings or amino derivatives thereof may be fully or partially acylated or completely deacylated. The completely or partially acylated glycosides are useful as defined intermediates for the synthesis of the deacylated materials.

The glycone moieties of the vitamin D glycosides can comprise up to 20 glycone units. Preferred, however, are those having fewer than 10, and most preferred are those having 3 or fewer than 3 glycone units. Specific examples are those containing 1 or 2 glycone units in the glycone moiety. Preferred are those with a β-glycoside linkage.

When more than one glycone unit is present on a single hydroxy group (*i.e*., di or polyglycosidic residues), the individual glycosidic rings may be bonded by 1-1, 1-2, 1-3, 1-4, 1-5 or 1-6 bonds, most preferably 1-2, 1-4 and 1-6. The linkages between individual glycosidic rings may be α or β.

The glycone moieties may comprise any glycone moiety known in the art. Preferred glycopyranosyl structures include glucuronic acid, glucose, mannose, galactose, gulose, allose, altrose, idose, or talose. Preferred furanosyl structures include those derived from fructose, arabinose, or xylose. Preferred diglycosides (*i.e*., glycone moieties with 2 glycone units) include sucrose, cellobiose, maltose, lactose, trehalose, gentiobiose, and melibiose. Preferred triglycosides (*i.e*., glycone moieties with 3 glycone units) include raffinose or gentianose. Preferred amino derivatives include N-acetyl-D-galactosamine, N-acetyl-D-glucosamine, N-acetyl-D-mannosamine, N-acetylneuraminic acid, D-glucosamine, lyxosylamine, D-galactosamine, and the like. Other preferred glycone moieties are described elsewhere in this application.

Examples of vitamin D glycosides of the present invention include vitamin D₃, 3β-(β-D-glucuronide); vitamin D₃, 3β-(β-D-glucopyranoside); vitamin D₃, 3β-(β-D-fructofuranoside); vitamin D₃, 3β-(galactoside); vitamin D₃, 3β-(β-maltoside); vitamin D₃, 3β-(β-lactoside); vitamin D₃, 3β-(β-trehaloside); vitamin D₃, 3β-raffinoside; vitamin D₃, 3β-gentiobioside; 1α-hydroxyvitamin D₃, 3β-(β-D-glucuronide); 1α-hydroxyvitamin D₃, 3β-(β-D-glucopyranoside); 1α-hydroxyvitamin D₃, 3β-(β-D-fructofuranoside); 1α-hydroxyvitamin D₃, 3β-(β-cellobioside); 1α-hydroxy-3β-(β-maltosyl)-vitamin D₃; 1α-hydroxy-3β-raffinosyl-vitamin D₃; 1α-hydroxy-3β-gentiobiosyl-vitamin D₃; 1α-(β-D-glucuronosyl)vitamin D₃; 1α-(β-D-glucopyranosyl)vitamin D₃; 1α-(β-D-fructofuranosyl)vitamin D₃; 1α-(β-galactosyl)vitamin D₃; 1α-(β-maltosyl)-vitamin D₃; 1α-(β-lactosyl)vitamin D₃; 1α-(β-trehalosyl)-vitamin D₃; 1α-raffinosyl-vitamin D₃; 1α-gentiobiosylvitamin D₃; 1α-(β-D-glucuronosyl)-25-hydroxyvitamin D₃; 1α-(β-D-glycopyranosyl)-25-hydroxyvitamin D₃; 1α-(β-D-fructofuranosyl)-25-hydroxyvitamin D₃; 1α-hydroxy-25(β-D-glucuronosyl)-vitamin D₃; 1α-hydroxy-25(β-D-fructofuranosyl)-vitamin D₃; 1α-hydroxy, 25-(β-glucopyranosyl)-vitamin D₃; 1α-hydroxy, 25-(β-maltosyl)-vitamin D₃; 1α-hydroxy, 25-(β-lactosyl)-vitamin D₃; 1α-hydroxy, 25-(β-trehalosyl)-vitamin D₃; 1α-hydroxy, 25-(raffinosyl)-vitamin D₃; 1α-hydroxy, 25-(gentiobiosyl)-vitamin D₃; 1α,24-dihydroxyvitamin D₃, 3β-(β-D-glucuronide); 1α,24-dihydroxyvitamin D₃, 3β-(β-D-glucopyranoside); 1α,24-dihydroxyvitamin D₃, 3β-(β-D-fructofuranoside); 1α-(β-D-glucuronosyl)-24-hydroxyvitamin D₃;1α-(β-D-glycopyranosyl)-24-hydroxyvitamin D₃; 1α-(β-D-fructofuranosyl)-24-hydroxyvitamin D₃; 1α-hydroxy-24-(β-D-fructofuranosyl)-vitamin D₃; 1α-hydroxy-24-(β-glycopryanosyl)-vitamin D₃; 1α-hydroxy,24-(β-maltosyl)-vitamin D₃; 1α-hydroxy,24-(β-lactosyl)-vitamin D₃; 1α-hydroxy,24-(β-trehalosyl)-vitamin D₃; 1α-hydroxy,24-(raffinosyl)-vitamin D₃; and 1α-hydroxy,24-(gentiobiosyl)-vitamin D₃. Most preferred are 1α,25-dihydroxyvitamin D_{3,}, 3β-(β-D-glucuronide); 1α,25-dihydroxyvitamin D₃, 3β-(β-D-glucopyranoside); 1α,25-dihydroxyvitamin D₃, 3β-(β-D-fructofuranoside); 1α,25-dihydroxyvitamin D₃, 3β-(galactoside); 1α,25-dihydroxyvitamin D₃, 3β-(β-maltoside); 1α,25-dihydroxyvitamin D₃, 3β-(β-lactoside); 1α,25-dihydroxyvitamin D₃, 3β-(β-trehaloside); 1α,25-dihydroxyvitamin D3, 3β-raffinoside; 1α,25-dihydroxyvitamin D₃, 3β-gentiobioside; 25-hydroxyvitamin D_{3,}, 3β-(β-D-glucuronide); 25-hydroxyvitamin D₃, 3β-(β-D-glucopyranoside); 25-hydroxyvitamin D₃, 3β-(β-D-fructofuranoside); 25-hydroxyvitamin D₃, 3β-(galactoside); 25-hydroxyvitamin D₃, 3β-(β-maltoside); 25-hydroxyvitamin D₃, 3β-(β-lactoside); 25-hydroxyvitamin D₃, 3β-(β-trehaloside); 25-hydroxyvitamin D₃, 3β-raffinoside; 25-hydroxyvitamin D₃, 3β-gentiobioside; 24,25-dihydroxyvitamin D_{3,}, 3β-(β-D-glucuronide); 24,25-dihydroxyvitamin D₃, 3β-(β-D-glucopyranoside); 24,25-dihydroxyvitamin D₃, 3β-(β-D-fructofuranoside); 24,25-dihydroxyvitamin D₃, 3β-(galactoside); 24,25-dihydroxyvitamin D₃, 3β-(β-maltoside); 24,25-dihydroxyvitamin D₃, 3β-(β-lactoside); 24,25-dihydroxyvitamin D₃, 3β-(β-trehaloside); 24,25-dihydroxyvitamin D₃, 3β-raffinoside; and 24,25-dihydroxyvitamin D₃, 3β-gentiobioside. All of the aforementioned derivatives can also be prepared with vitamin D₂, D₄, or D₅.

Non-limiting examples of vitamin D sulfates include each of the compounds described in the preceding paragraph but comprising a sulfate group in place of the glycone moiety.

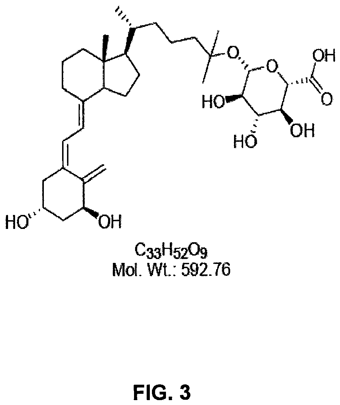

Preferred prodrugs comprise those wherein the glycone or sulfate moiety is attached to the carbon at the 25 position, such as the vitamin D glucuronide shown in FIG. 3. The glucuronide moiety in FIG. 3 can be substituted with a sulfate or any other glycone described herein, and the vitamin D-drug moiety shown in FIG. 3 can be replaced with any vitamin D drug described herein that accommodates a pro group at the 25 position.

The vitamin D prodrugs described herein are prepared or obtained according to methods which are well known to those of ordinary skill in the art. For example, the glycosidic derivatives of the aforementioned compounds may be obtained according to Holick, U.S. Pat. No. 4,410,515. The vitamin D glycosyl orthoester compounds may be obtained according to U.S. Pat. No. 4,521,410. The 5,6-epoxy derivatives of vitamin D₃ are obtained as described in Jpn. Kokai Tokyo Koho JP 58,216,178 [83,216,178], Dec. 15, 1983. The fluoro derivatives are made or obtained as described in Shiina, et al., Arch. Biochem. Biophys. 1983 220:90. Methods for preparing the 20-and 22-oxa vitamin D derivatives are disclosed by Abe, J., et al., Vitamin D Molecular, Cellular and Clinical Endocrinology, p. 310-319, Walter de Gruyter & Co., Berlin (1988). U.S. Pat. No. 4,719,205 to DeLuca et al. discloses methods for the preparation of 22,23-cis-unsaturated, 1-hydroxyvitamin D compounds. U.S. Pat. No. 4,634,692 to Partridge et al. discloses methods for the preparation of 1,25-dihydroxy-24 (R or S)-fluorovitamin D. Japanese Patent Application, publication no. J55 111-460, discloses methods for the preparation of 24,24-difluoro-25-hydroxyvitamin D₃.

Any animal which experiences hyperproliferative, autoimmune, or infectious diseases and which may benefit from the vitamin D drugs described herein may be treated with the vitamin D prodrugs according to the present invention. Preferred animals are mammals, and most preferred are humans.

In some versions of the invention, a vitamin D prodrug described herein is administered to an individual to treat a tumour, cancer, or neoplastic growth.

In a more specific version, a vitamin D glycoside, such as a vitamin D glucuronide, is administered to an individual to treat a tumour, cancer, or neoplastic growth, with β-glucuronidase serving as a cleaving enzyme.

The physiological function of β-glucuronidase is the degradation of glucuronic acid-containing glucosaminoglycans (like heparan sulfate, chondroitin sulfate, and dermatan sulfate) (Paigen K., Prog Nucleic Acid Res Mol Biol 1989 37:155-205). The endogenous enzyme is located in lysosomes and is therefore not available for cleaving under normal circumstances. In addition, β-glucuronidase leaking out of normal cells is rapidly internalized via the mannose-6-phosphate (M6P) receptor on the cell surface. The optimum pH of β-glucuronidase is approximately 5.5, which corresponds to its natural acid environment in lysosomes. Similar to secreted proteins, all natural lysosomal proteins have a leader sequence but bind in the endoplasmic reticulum (ER) to the mannose-6-phoshate receptor, which identifies these proteins for translocation to the lysosomes. If the expression of lysosomal proteins exceeds the capacity of this mechanism (as determined by the numbers of mannose-6-phoshate receptors in the ER) the protein will be secreted.

The vitamin D glycosides for use in the present invention are hydrophilic and do not easily enter living cells. This extracellular localization prevents its conversion to the cleaved vitamin D pro-drug or related compounds in the vicinity of non-diseased cells, which normally maintain β-glucuronidase or other glycosidases intracellularly within lysosomes. By contrast, the cleaved vitamin D-drug moieties are lipophilic and are rapidly taken up by surrounding cells. This limits their entrance into the circulation in the active form.

In many tumour cells, β-glucuronidase is present at higher levels than in the surrounding normal tissue cells. Additionally, in contrast to normal tissue, tumoural β-glucuronidase is in part localized extracellularly. Both higher expression levels and extracellular localization of tumoural β-glucuronidase aid in selectively releasing the free vitamin D drug from the hydrophilic glucuronide in the area of the tumour.

In addition to higher expression levels and extracellular localization of β-glucuronidase in tumours, tumours often have a higher local concentration of β-glucuronidase due to necrotic tissue associated with the tumour. The necrotic tissue includes areas where tumour cells, neutrophils, and macrophages have died and released their intracellular contents, including the β-glucuronidase normally contained in lysosomes. In cases where a tumour does not have a significant amount of necrotic areas or enhanced cleavage of the glycoside is desired, a patient may be pre-treated with conventional chemotherapy to induce an initial destruction of cells to generate necrotic tissue. This will cause an additional release of endogenous β-glucuronidase and result in an amplification of the cleavage of the vitamin D glycosides.

Prodrugs such as vitamin D sulfates or vitamin D glycosides other than vitamin D glucuronide may also be used to treat cancer. However, in some versions of the invention, enzymes that cleave such vitamin D prodrugs may need to be delivered to the cancerous target tissues (see below).

In one method of treating cancer, the vitamin D prodrug is injected at doses from 0.001 milligram to 0.5 grams and at dosing intervals based on the response to the therapy and levels of vitamin D prodrug products in the serum. The dose is advantageously in the range of 0.005 µg-500 mg/m² with dose cycles of tumour pH modulation and vitamin D prodrug administration each day for up to 20 days, depending on the level of non-specific activation as measured by the appearance of vitamin D prodrug products in the serum. This cycle of therapy is repeated a number of times (3-10 times) as required.

With regard to vitamin D glucuronidases, the specificity of targeting and activation of the vitamin D glucuronidase in the area of a tumour can be enhanced by the use of glucose and alkalinization to increase the differences in pH between the tumour and the normal tissues. The use of glucose allows the tumour pH to be lowered significantly, and the use of a base such as sodium bicarbonate allows the urine pH and other areas of normal tissue to remain at a pH in the range of 7.4. The lowering of the tumour pH can be as much as 0.5 pH units in some cases (Cancer Res. 1989 49:4373-4384). The decrease in pH in the tumour relative to non-cancerous tissue renders the β-glucuronidase more active in the tumour.

To adjust the pH of tumours in a patient prior to treatment with the vitamin D glucuronides, the patient is typically first given juices and asked to empty his or her bladder. This is followed by a dose of 100 g of glucose. After 30 min to 2 hrs, the patient then receives a drip which delivers 10% glucose and 60 milliequivalents of sodium bicarbonate. This drip delivers up to 1 liter over one hour. At 30 min into the drip, the patient empties his or her bladder to determine the effectiveness of the therapy in causing alkalinization of the urine. Alkalinization is also achieved by the use of inhibitors of carbonic anhydrase (i.e., acetazolamide) in combination with bicarbonate to achieve a more prolonged effect. The alkalinization protocol can be optimized or adjusted accordingly.

The treatment with the vitamin D glucuronide is initiated when it has been determined that the glucose and bicarbonate drip has achieved alkalinization of the urine. The analysis of the 30 min urine sample should show a pH above 7.4. The vitamin D glucuronide is typically given as an infusion in order to maintain a sustained level of drug in the blood for a period of one hour or more. Alternatively, the vitamin D glucuronide is given as a bolus IV. The dose of the vitamin D glucuronide is a maximum of 500 mg/m² per treatment round but can be fractionated into multiple doses. Patients may be eligible for further treatment based on the indications of toxic side effects. Treatment rounds occur at intervals of 1-3 weeks. This treatment protocol can be optimized or adjusted accordingly.

Regardless of the type of prodrug administered, patients are monitored for hypercalcemia and symptomatic hypercalcemia according to the parameters outlined in Table 1. These parameters include monitoring adequate organ function, including hematological function (white cell count, platelet count), hepatic function (bilirubin, aspartate amino transferase, alanine aminotransferase), and renal function (creatinine levels). This data is useful as a basis for controlling dose and intervals during treatment.

In addition to, or in place of, adjusting pH, several other techniques may be used to increase both the specificity and the effectiveness of the vitamin D prodrugs in target sites such as cancerous tissue. One method involves delivering enzymes having the appropriate glycosidase or sulfatase activity to the target sites.

One such technique is gene-directed enzyme-pro-drug therapy (GDEPT). In GDEPT, bacteria or viruses are used to deliver DNA coding the enzymes to the tumour cells. The target cells then begin producing and in some cases secreting the preferred enzyme in large amounts. This enhances the cleavage of the therapeutic compound from the pro moiety at the site of the tumour (Huber B E et al. Cancer Res. 1993 53:4619-4626).

In one example of GDEPT, a retroviral vector which contains DNA encoding an enzyme which is capable of activating a vitamin D prodrug of the invention is generated. This viral vector is then targeted via the selective nature of the infectious agent for dividing cells or via the selective expression systems within the cell. For example, transcription of the DNA encoding the glycosidic enzyme may be controlled by a promoter recognized only by target cells, or translation of the DNA transcript encoding the glycosidic enzyme may be controlled by factors expressed only by the target cells. Viruses other than retroviral vectors can be used in this targeting approach, including adenovirus, fowlpox, or Newcastle disease virus. The delivery of the virus can be directed through the use of an infectious particle which optionally has been engineered to have a selective tissue tropism (i.e., by inclusion of antibody binding domains). In an alternative method, the virus is targeted by the use of other vehicles such as liposomes in either a targeted (by binding moieties, *i.e*., antibodies) or untargeted fashion (Bichko V et al. J Virology. 1994 68:5247-5252).

The targeting of the appropriate enzyme gene to achieve the selective activation of the vitamin D prodrugs of the invention can also be achieved using other organisms which show tropisms for tissues and organs.

The targeting and delivery of enzyme genes to activate vitamin D prodrugs can also occur via the delivery of DNA by non-viral mechanisms such as liposomes. This may be achieved, for example, by making use of transmembrane domains of membrane binding proteins or binding domains of antibodies, etc., within the liposome.

In addition, transformed cells may be used to target the delivery of enzyme activity to the site of therapy. See Cancer Immunol Immunother. 1994 Maj; 38(5):299-303, Cancer, 1994, March 15; 73(6)1731-7.

The glycosidases or sulfatases are preferably expressed in these virally based or non-virally based targeting systems in a form in which the enzymes do not diffuse away from the tumour or other target site, such as by fusing the enzymes to a cell-surface receptor.

Another technique of increasing β-glucuronidase or other glycosidase activity at a target site is known as antibody-directed enzyme-pro-drug therapy (ADEPT). In this technique, the enzyme of interest (glycosidase, sufatase) is bonded to an antibody that is directed against a particular type of target cell. Antibodies against virtually any type of cell are commercially available or can be made by methods known in the art (Sambrook et al., In: Molecular Cloning: A Laboratory Manual, 3rd ed., Cold Spring Harbor Laboratory Press (2001)). The antibody thus specifically delivers higher levels of the activating enzyme to the surface of the target cells. Examples of targeting antibodies which can be used to treat cancer are OncoScint® (Cytogen Corp Princeton N.J.), which is capable of achieving in some cases tumour:normal tissue ratios of greater than 20:1 (Stern H, et al. Cancer Investigation 1993, 11(2) 129-134), and CA125, BR96, B72.3, CC49, Col1, 17-1A, and 16.88, which include both mouse, humanized and human antibodies (Siddiki B et al. Int J Cancer. 1993 54:467-474; Weiner L M et al. J Immunotherapy. 13:110-116; Muraro R, et al. Cancer Res. 1985 45:5769-5780; Colcher D et al. Cancer Res. 1988 48:4597-4603; Jager R D, et al. Seminars in Nuclear Medicine. 1993 XXIII:165-179). See also U.S. patent application Ser. Nos. 07/773,042 and 07/919,851. These antibodies are linked by a chemical linkage or via the construction of genetic fusions. These molecules are dosed prior to the administration of vitamin D prodrugs of the invention.

An antibody-enzyme fusion protein is administered at up to 1 µM in various dosing schedules, but typically in the range 0.001-200 mg per dose as a single dose which may be infused over a period of time from 10 min to 24 hr. The dose of antibody-enzyme can also be given in multiple dose injections. After antibody-enzyme infusion, the levels of enzyme and the antibody titers are periodically measured. The typical time allowed for clearance is from 1 to 14 days. When the levels of prodrug-cleaving enzyme have reached a level that optimizes activation of the prodrug at targeted sites, the vitamin D prodrug is administered. The vitamin D prodrug is injected at doses up to 5 grams and at dosing intervals based on the response to therapy and levels of prodrug products in the serum. Advantageously, the vitamin D prodrug dose is in the range of 0.005 µg-500 mg/m² of prodrug with doses each day or intermittently for up to 20, 30, 40, or more days. The rate of administration will vary depending on the level of non-specific activation as measured by the appearance of prodrug products in the serum and monitoring of the dose limiting toxicity using HPLC analysis of extracted blood samples and serum chemistry analysis.

The glycosidase or sulfatase enzymes used for ADEPT and GDEPT may be derived from any organism. Preferred versions include those having bacterial, yeast, or viral origin.

The prodrugs and treatments described herein may be used to treat tumours, cancers, or neoplastic growth in the prostate, breast, intestine, colon, lung, pancreas, endometrium, bone marrow, blood cells, cervix, thyroid, ovaries, skin, retina, kidney, connective tissue (bone, cartilage, and fat), epithelia, and bladder, among other tissues. Non-limiting examples of specific cancers that can be treated include squamous cell carcinoma, myeloid leukemia, retinoblastoma, sarcomas of the soft tissues, renal cell carcinoma, myeloid and lymphocytic leukemia, medullary thyroid carcinoma, melanoma, and multiple myeloma. Any neoplastic disease now known or discovered that are sensitive to vitamin D can be treated with the vitamin D prodrugs described herein.

Also disclosed is the use of vitamin D prodrugs to treat infection, such as bacterial infection. Bacterial infections that can be treated with vitamin D prodrugs include, without limitation, infections with *Streptococci*; *Staphylococci*, such as *Staphylococcus aureus*; *Escherichia*, including *E. coli*; *Mycobacteria*, including *Mycobacterium bovis*, and *Mycobacterium tuberculosis*; *Clostridium*, such as *Clostridium perfringens* and *Clostridium difficile*; *Campylobacter jejuni*; *Yersinia*; *Salmonella*; and *Shigella.*

The vitamin D prodrugs of the invention may be used in treating bacterial infections in which the bacteria involved have a specific glycosidase or sulfatase activity. Non-limiting examples of such bacteria include *Streptococci*, *Staphylococci*, and *E. coli*. Glycosidase or sulfatase activity of other bacteria (or other target sites) is easily determined by methods known in the art (see, *e.g*., U.S. Pat. 5,891,620) and as shown in the examples. Treatment is as described above for cancer or as described elsewhere herein.

The vitamin D prodrugs may also be used in treating bacterial infections in which the bacteria do not exhibit glycosidase activity but are within or in the vicinity of sites having glycosidase activity. For example, endogenous β-glucuronidase is present in sites of infection/inflammation where the enzyme has been released as bacteria, neutrophils, and/or macrophages die. The amount of glycosidase activity present is sufficient for targeting the cleaved form of the vitamin D glycoside to the sites of infection. In addition, the intestinal tract, and in particular the lower intestinal tract, possesses both β-glucuronidase and sulfatase activity sufficient for producing vitamin D-dependent effects in the intestine. See the examples that follow.

The vitamin D prodrugs of the invention may also be used in treating infections in which neither the bacteria nor sites in the vicinity of the bacteria produce a suitable glycosidase. In such a case, the targeting techniques used as described above for cancer may be used for treatment of infection. These targeting techniques include but are not limited to ADEPT and GDEPT. For targeting of specific bacteria by GDEPT, bacteria-specific vectors, such as phages, and expression systems of specific bacteria are well known in the art. For targeting of specific bacteria by ADEPT, antibodies that specifically recognize the specific bacteria are also well known in the art and are commercially available. Otherwise, antibodies directed against a particular bacterium can be made (Sambrook et al., In: Molecular Cloning: A Laboratory Manual, 3rd ed., Cold Spring Harbor Laboratory Press (2001)).

Although hyperacidification with glucose is not required for vitamin D prodrug treatment of infection, it may be used. Furthermore, alkalinization may be carried out to reduce non-specific activation glucuronide-containing prodrugs, as described above. Bicarbonate drips or drug treatments (e.g., acetazolamide) can be used. Having established this alkalinization, the vitamin D prodrug is given via the bicarbonate drip or by intravenous injection in a suitable vehicle. Alkalinization can also be achieved by oral bicarbonate.

Another version of the invention comprises the use of vitamin D prodrugs to treat inflammatory, autoimmune, and Th1-related diseases. Examples of such diseases that may be treated with the vitamin D prodrugs include but are not limited to type 1 diabetes, multiple sclerosis, inflammatory bowel disease, alopecia areata, autoimmune cardiopathy, and psoriasis. The vitamin D prodrugs described herein can be used to treat any disease now known or discovered to be sensitive to vitamin D. The treatment of these diseases occurs as described above for cancer or as described elsewhere herein and may include the use of GDEPT and ADEPT, the latter with commercial or generated antibodies directed against the particular target site.

Treatment of any of the hyperproliferative diseases, infections, or inflammatory, autoimmune, or Th1-related diseases described herein can occur wherein the treated cells or tissues directly express the appropriate cleaving enzyme, are in the vicinity of such enzyme activity, or are targeted to exhibit such enzyme activity by, *e.g*., GDEPT or ADEPT.

Treatment of the diseases in the present invention preferably occur without inducing hypercalcemia or symptoms of hypercalemia. As shown in the examples, the hypercalcemic activity of the vitamin D prodrugs is of from about 4-fold to about 18-fold less than their non-prodrug counterparts. In addition, the vitamin D prodrugs are as effective as the non-prodrug counterparts in producing therapeutic effects in target tissues.

Accordingly, some versions of the invention include treating a vitamin D-sensitive disease with a vitamin D prodrug without inducing hypercalcemia. Various versions of the invention comprise treating the vitamin D-sensitive disease while keeping calcemia to a level of grade 3 or lower, grade 2 or lower, grade 1 or lower or grade 0, as characterized in Table 1.

In other versions of the invention, the vitamin D prodrugs may induce a degree of hypercalemia but with symptoms that are reduced with respect to the non-prodrug counterparts. Accordingly, various versions of the invention comprise treating the vitamin D-sensitive disease while controlling hypercalcemia symptoms at a toxicity grade of grade 3 or lower, grade 2 or lower, grade 1 or lower, or grade 0, as characterized in Table 1. Some versions of the invention comprise treating the vitamin D-sensitive disease without inducing severe symptomatic hypercalcemia (*i.e*., hypercalcemia with symptoms characteristic of grades 3 or 4). Amounts of the vitamin D prodrugs that do or do not produce such effects when administered are described herein as "non-hypercalcemia-inducing amount," "non-grade-O-hypercalcemia-inducing amount," "non-severe-symptomatic-hypercalcemia-inducing amount," etc. The reduced hypercalcemic effect of the vitamin D prodrugs may result from any number of factors, including but not limited to activation at the target site and limited intestinal absorption (*e.g*., see examples).

The examples show several unexpected results of orally administering vitamin D prodrugs. Namely, the examples show that vitamin D prodrugs, specifically vitamin D glycosides, are systemically absorbed much less efficiently than their non-glycoside counterparts. The examples also show that the lower intestinal tract (*i.e*., the ileum and colon) but not the upper intestinal tract (*i.e*., the duodenum) comprises glycosidase activity sufficient to cleave vitamin D glycosides therein. It is predicted that vitamin D sulfates are also systemically absorbed much less efficiently than their non-glycoside counterparts and that the lower intestinal tract but not the upper intestinal tract comprises sulfatase activity sufficient to cleave vitamin D sulfates therein. These characteristics render the vitamin D prodrugs particularly useful for treating vitamin D-sensitive diseases of the intestinal tract, particularly the lower intestinal tract, in a manner that renders a patient less susceptible to hypercalcemia or symptoms resulting therefrom. Without being limited by mechanism, it is thought that bacteria and inflammatory cells in the ileum and large intestine serve as sources of the glycosidases and sulfatases that activate the vitamin D glycoside and thus target the cleaved (free) form of the vitamin D drug to these regions of the intestine.

Accordingly, some versions of the invention comprise treating vitamin D-sensitive intestinal diseases. Such diseases can include any vitamin D-sensitive disease or condition included in or confined to the intestine or portions thereof, including the jejunum, the ileum, and the colon (ascending colon, transverse colon, and sigmoid colon). Vitamin D-sensitive intestinal diseases that can be treated with vitamin D prodrugs include neoplastic diseases of the intestine, infections of the intestine, and autoimmune diseases of the intestine, among others. Non-limiting, exemplary neoplastic diseases of the intestine that can be treated with vitamin D prodrugs include colorectal cancer or other cancers of the intestine. Non-limiting, exemplary infections of the intestine that can be treated with vitamin D prodrugs include infections with *Staphylococcus*, such as *Staphylococcus aureus*; *Clostridium*, such as *Clostridium perfringens* and *Clostridium difficile*; *Escherichia*, such as *E. coli*; *Campylobacter*, such as *Campylobacter jejuni*; *Yersinia*; *Salmonella*; and *Shigella.* Non-limiting, exemplary autoimmune diseases that can be treated with vitamin D prodrugs include irritable bowel syndrome, Crohn's disease, and celiac disease. Other vitamin D-sensitive intestinal diseases that can be treated with vitamin D prodrugs include inflammatory bowel diseases, whether having an autoimmune etiology or not, such as ulcerative colitis, and diseases such as pseudomembranous colitis.

Other versions include selectively treating a vitamin D-sensitive intestinal disease with a vitamin D prodrug. As used herein with reference to treating vitamin D-sensitive intestinal diseases, "selectively treating" means treating the disease wherein the vitamin D prodrug or cleaved (free) vitamin D-drug moieties derived therefrom are substantially confined to the intestinal tract and are substantially inhibited from being absorbed systemically. In specific versions of selectively treating a vitamin D-sensitive intestinal disease, the plasma levels of the free vitamin D-drug moiety derived from the vitamin D prodrug does not increase to more than about 14-fold, 10-fold, 7.5-fold, or 5-fold more than baseline plasma vitamin D levels at any point after administration of the vitamin D prodrug. "Baseline plasma vitamin D levels" refers to the level of active vitamin D (*e.g*., 1,25-dihydroxyvitamin D₃, etc.) circulating in a patient's plasma prior to treatment with the vitamin D prodrug.

Other versions of the invention include selectively treating a vitamin D-sensitive intestinal disease in the lower intestine. As used herein with reference to treating a vitamin D-sensitive intestinal disease in the lower intestine, "selectively treating" means treating the disease wherein the vitamin D prodrug or cleaved (free) vitamin D-drug moieties derived therefrom are substantially confined to the intestinal tract and are substantially inhibited from being absorbed systemically, and further wherein the vitamin D prodrug is substantially cleaved only upon reaching the lower intestinal tract, such as the ileum and/or colon. In specific versions of selectively treating a vitamin D-sensitive intestinal disease in the lower intestine, the plasma levels of the free vitamin D-drug moiety derived from the vitamin D prodrug does not increase more than about 14-fold, about 10-fold, about 7.5-fold, or about 5-fold more than baseline plasma vitamin D levels at any point after administration of the vitamin D prodrug. In other specific versions of selectively treating a vitamin D-sensitive intestinal disease in the lower intestine, a proportion of at least about 10%, about 20%, about 30%, about 40%, or about 50% of the initially administered vitamin D prodrug is cleaved only upon reaching the lower intestine, or at least portions of the intestine downstream of the duodenum.

Treatment of vitamin D-sensitive intestinal diseases is preferably performed via oral administration of a vitamin D prodrug. However, rectal administration is also acceptable. For oral administration, enteric coatings may optionally encapsulate the vitamin D prodrug. The enteric coatings break down in the lower intestinal tract and further aid in the selective delivery of the vitamin D prodrug to this region. Because glycosidase and sulfatase activity is confined to the lower intestinal tract, however, enteric coatings for the vitamin D prodrugs are not required for selective targeting of the vitamin D drugs to the lower intestinal tract. Regardless of the mechanism, targeting the vitamin D drug to the lower intestine reduces the amount of the drug absorbed, thereby reducing the risk of inducing severe hypercalcemia.

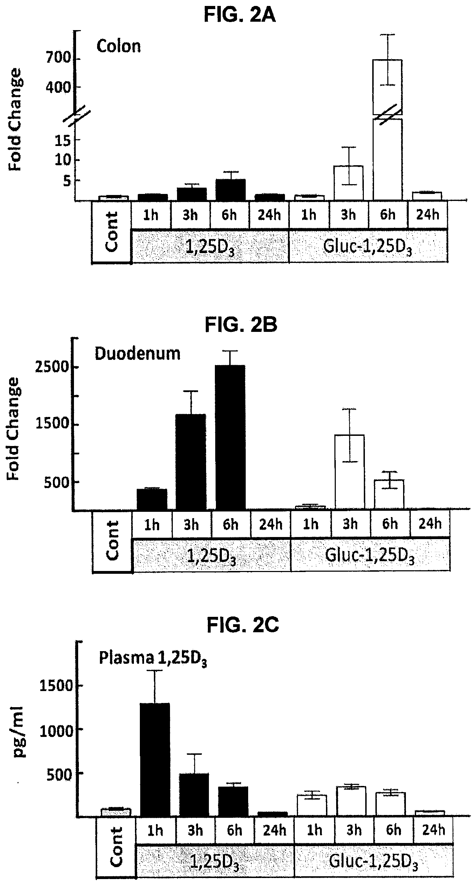

Another unexpected result of orally administering vitamin D prodrugs shown in the examples is that the plasma level of the cleaved (free) vitamin D-drug moiety resulting from a single dose of a vitamin D prodrug comprising it does not spike and is relatively constant over the course of about 6 hours. This is contrasted with direct administration of the non-glycosidated and non-sulfated forms of the vitamin D drug, which causes a drastic spike in the plasma level of the drug one hour after administration, and which is followed by a sharp drop in levels at 3-and 6-hour intervals thereafter (see FIG. 2C). Thus, oral administration of vitamin D prodrugs, even without being packaged in sustained-release capsules or other specific sustained-release formulations, are unexpectedly useful in systemically treating vitamin D-sensitive diseases by raising the plasma level of a vitamin D drug to a consistent level over time. Specific versions include raising plasma level of a free vitamin D-drug moiety derived from a vitamin D prodrug to levels that remain within about ±70%, about ±60%, about ±50%, about ±40%, about ±30% of any given level over the course of about 3, 4, or 5 hours following a single oral dose of the vitamin D prodrug. The vitamin D prodrug used in such versions is preferably comprised within a composition devoid of conventional sustained-release formulations. Conventional sustained-release formulations are also commonly known in the art as sustained-action, extended-release, time-release, timed-release, controlled-release, modified-release, or continuous-release formulations. Conventional sustained-release formulations typically embed the active ingredient in a matrix of insoluble substances such as acrylics or chitin or are enclosed in a polymer-based tablet with a laser-drilled hole on one side and a porous membrane on a second side.

Oral administration of the vitamin D prodrugs can be used for systemically treating diseases by minimally and consistently increasing the plasma concentration of a vitamin D drug for extended periods of time, such as 3, 4, or 5 hours at a time. The plasma concentration of the free vitamin D-drug moiety derived from the vitamin D prodrug can be increased to a level of no more than about 14-fold, about 10-fold, about 7.5-fold, or about 5-fold more than baseline plasma vitamin D levels at any point after administration of the vitamin D prodrug. Administering a dose every 3, 4, or 5 hours can be performed to maintain the consistent plasma level of the vitamin D-drug moiety. Oral administration of the vitamin D prodrugs is acceptable but not preferred for treating diseases requiring large, acute, systemic doses of a vitamin D drug.

The invention includes treating a subject with vitamin D prodrugs comprising an inhibitor of vitamin D 24-hydroxylase as the vitamin D-drug moiety as defined in claim 1. The inhibitor is preferably a competitive inhibitor and is also preferably an inactive vitamin D drug. Inactive vitamin D drugs that are inhibitors of the vitamin D 24-hydroxylase may include any vitamin D analog that does not have a hydroxyl group at the C-1 position. Such inhibitors are also preferably hydroxylated at the C-25 and/or the C-24 positions. Examples of competitive inhibitors of the vitamin D 24-hydroxylase include, without limitation, glycosides and sulfates of 25-hydroxyvitamin D or 24,25-dihydroxyvitamin D. These compounds may be in the vitamin D₂, D₃, D₄, or D₅ forms. When activated by the relevant enzymes in targeted tissues, these vitamin D 24-hydroxylase-inhibiting prodrugs increase the local concentration of cleaved (freed) vitamin D-drug moieties or other vitamin D compounds by inhibiting their degradation.

More specifically, the invention encompasses the use of β-glucuronides of vitamin D drugs that competitively inhibit the vitamin D 24-hydroxylase. These are used to deliver high and effective doses of inhibitors of the vitamin D 24-hydroxylase to target cells expressing β-glucuronidase activity. For example, when 25-β-glucuronide-25-hydroxyvitamin D₃ is administered orally, the β-glucuronidase produced by bacteria residing in the lower intestine hydrolyses the β-glucuronide bond, causing local levels of 25-hydroxyvitamin D to increase in the lower intestine. The 25-hydroxyvitamin D can competitively inhibit vitamin D 24-hydroxylase, prolonging the half life of 1,25-dihydroxyvitamin D in that area. This potentiates the action of 1,25-dihydroxyvitamin D on cells of the ileum and colon. This allows therapeutic effects with a lower dose of vitamin D drugs, which reduces the risk of hypercalcemia.

The vitamin D 24-hydroxylase is upregulated within many cancerous cells (Cross HS. Nutr Rev. 2007 Aug;65(8 Pt 2):S108-12). It is also upregulated in inflammatory bowel disease (Liu et al., Endocrinology. 2008 149(10):4799-4808). Because the vitamin D 24-hydroxylase is greatly upregulated in many of these cells, the amount of vitamin D drug required to effectively treat the cells is increased. This also tends to increase the risk of hypercalcemia developing during treatment. The use of vitamin D prodrugs comprising competitive inhibitors for the vitamin D 24-hydroxylase reduces the rate at which 1,25-dihydroxyvitamin D is catabolized and lowers the effective therapeutic dose of vitamin D or analogs thereof.

Any treatment of any disease described herein may comprise administering a vitamin D prodrug comprising an active vitamin D drug as the vitamin D-drug moiety, a vitamin D drug comprising a 24-hydroxylase-inhibiting vitamin D-drug moiety, or both simultaneously or in sequence.

The 24-hydroxylase activity of many potential target tissues can be downregulated in the patient by administration of calcitonin. See Beckman et al., Endocrinology. 1994 135(5): 1951-5. This treatment can prolong the activity of both the vitamin D prodrugs producing the therapeutic benefits and the vitamin D prodrugs intended to inhibit the 24-hydroxylase. Calcitonin also can reduce plasma calcium levels by reducing osteoclast activity and increasing urinary calcium excretion which may also allow higher dosage of the vitamin D prodrugs without risk of developing hypercalcemia.

Some versions of the invention include administering, by any method, a vitamin D prodrug comprising a 25-hydroxylated vitamin D compound as the vitamin D-drug moiety. Suitable vitamin D-drug moieties in this version include, without limitation, 25-hydroxylated vitamin D compounds such as 25-hydroxyvitamin D₂, 25-hydroxyvitamin D₃, 25-hydroxyvitamin D₄, and 25-hydroxyvitamin D₅. Administering 25-hydroxylated vitamin D prodrugs provides target sites with substrate for local (autocrine) production of 1,25-dihydroxyvitamin D. In one version of the invention, oral administration of 25-hydroxylated vitamin D prodrugs delivers high concentrations of 25-hydroxyvitamin D in the vicinity of cells in the ileum and colon to provide substrate for 1,25-dihydroxyvitamin D production within these cells.

The present invention further includes pharmaceutical compositions suitable for use in any of the methods described herein. Such compositions may include any one or more vitamin D prodrugs that comprise any vitamin D-drug moiety and any pro moiety described herein.

Pharmaceutical compositions for use in the treatments described herein comprise one or more vitamin D prodrugs or pharmaceutically-acceptable salts thereof, optionally in combination with an acceptable carrier and optionally in combination with other therapeutically-active ingredients or inactive accessory ingredients. The carrier must be pharmaceutically-acceptable in the sense of being compatible with the other ingredients of the formulation and not deleterious to the recipient. The pharmaceutical compositions include those suitable for oral, topical, inhalation, rectal or parenteral (including subcutaneous, intramuscular and intravenous) administration.

The formulations may conveniently be presented in unit dosage form and may be prepared by any of the methods well known in the pharmaceutical arts. The term "unit dosage" or "unit dose" is denoted to mean a predetermined amount of a vitamin D prodrug sufficient to be effective for treating an indicated activity or condition. Making each type of pharmaceutical composition includes the step of bringing a vitamin D prodrug into association with a carrier and one or more optional accessory ingredients. In general, the formulations are prepared by uniformly and intimately bringing a vitamin D prodrug into association with a liquid or solid carrier and then, if necessary, shaping the product into the desired unit dosage form.

Formulations of the present invention suitable for oral administration may be presented as discrete units such as capsules, cachets, tablets, boluses or lozenges, each containing a predetermined amount of a vitamin D prodrug; as a powder or granules; or in liquid form, *e.g*., as an oil, aqueous solution, suspension, syrup, elixir, emulsion, dispersion, or the like.

A tablet may be made by compression or molding, optionally with one or more accessory ingredients. Compressed tablets may be prepared by compressing in a suitable machine a vitamin D prodrug in a free-flowing form, *e.g*., a powder or granules, optionally mixed with accessory ingredients, *e.g*., binders, lubricants, inert diluents, surface-active or dispersing agents. Molded tablets may be made by molding in a suitable machine a mixture of a powdered vitamin D prodrug with any suitable carrier.

Formulations suitable for parenteral administration by injection or otherwise conveniently comprise a sterile preparation of a vitamin D prodrug in, for example, water, saline, a polyethylene glycol solution, and the like, which is preferably isotonic with the blood of the recipient.

Useful formulations also comprise concentrated solutions or solids containing a vitamin D prodrug, which upon dilution with an appropriate solvent give a solution suitable for parenteral administration.

Preparations for topical or local applications comprise aerosol sprays, lotions, gels, ointments, suppositories *etc*., and pharmaceutically-acceptable vehicles therefor such as water, saline, lower aliphatic alcohols, polyglycerols such as glycerol, polyethylene glycerol, esters of fatty acids, oils and fats, silicones, and other conventional topical carriers. In topical formulations, the subject compounds are preferably utilized at a concentration of from about 0.001 % to 5.0% by weight.

Compositions suitable for rectal administration comprise a suppository, preferably bullet-shaped, containing a vitamin D prodrug and pharmaceutically-acceptable vehicles therefor such as hard fat, hydrogenated cocoglyceride, polyethylene glycol, and the like. In suppository formulations, the subject compounds are preferably utilized at concentrations of from about 0.000001% to 1% by weight.

Compositions suitable for rectal administration may also comprise a rectal enema unit containing a vitamin D prodrug and pharmaceutically-acceptable vehicles therefor such as 50% aqueous ethanol or an aqueous salt solution which is physiologically compatible with the rectum or colon. The rectal enema unit consists of an applicator tip protected by an inert cover, preferably comprised of polyethylene, lubricated with a lubricant such as white petrolatum and preferably protected by a one-way valve to prevent back-flow of the dispensed formula, and of sufficient length, preferably two inches, to be inserted into the colon via the anus. In rectal formulations, the subject compounds are preferably utilized at concentrations of from about 0.000001 % to about 1% by weight.

Useful formulations also comprise concentrated solutions or solids containing a vitamin D prodrug which upon dilution with an appropriate solvent, preferably saline, give a solution suitable for rectal administration. The rectal compositions include aqueous and non-aqueous formulations which may contain conventional adjuvants such as buffers, bacteriostats, sugars, thickening agents and the like. The compositions may be presented in rectal single dose or multidose containers, for example, rectal enema units.

Preparations for topical or local surgical applications for treating a wound comprise dressings suitable for wound care. In both topical and local surgical applications, the sterile preparations of a vitamin D prodrug are preferably utilized at concentrations of from about 0.001% to 5.0% by weight applied to a dressing.

Compositions suitable for administration by inhalation include formulations wherein the vitamin D prodrug is a solid or liquid admixed in a micronized powder having a particle size in the range of about 5 microns or less to about 500 microns or liquid formulations in a suitable diluent. These formulations are designed for rapid inhalation through the oral passage from conventional delivery systems such as inhalers, metered-dose inhalers, nebulizers, and the like. Suitable liquid nasal compositions include conventional nasal sprays, nasal drops and the like, of aqueous solutions of a vitamin D prodrug.

In addition to the aforementioned ingredients, the formulations of this invention may further include one or more optional accessory ingredient(s) used in the art of pharmaceutical formulations, *e.g*., diluents, buffers, flavoring agents, colorants, binders, surface-active agents, thickeners, lubricants, suspending agents, preservatives (including antioxidants) and the like.

The amount of a vitamin D prodrug required to be effective for any indicated condition will, of course, vary with the individual mammal being treated and is ultimately at the discretion of the medical or veterinary practitioner. The factors to be considered include the condition being treated, the route of administration, the nature of the formulation, the mammal's body weight, surface area, age and general condition, and the particular compound to be administered. In general, a suitable effective dose is in the range of about 0.001 ng to about 20 µg/kg body weight per day, preferably in the range of about 0.01 to about 700 ng/kg per day or about 100 ng/kg per day, calculated as the non-salt form. The total daily dose may be given as a single dose, multiple doses, e.g., two to six times per day, or by intravenous infusion for a selected duration. Dosages above or below the range cited above are within the scope of the present invention and may be administered to the individual patient if desired and necessary.

In general, the pharmaceutical compositions of this invention contain from about 0.05 µg to about 1.5 g vitamin D prodrug per unit dose and, preferably, from about 0.75 µg to about 0.1 mg per unit dose. If discrete multiple doses are indicated, treatment might typically be 0.01 mg of a vitamin D prodrug, given from two to four times per day.

Alternatively, the vitamin D prodrug may be administered at any level to generate a concentration of between about 1 pM and 1 µM, preferably between about 10 pM and 100 nM, and more preferably between about 100 pM and 10 nM local concentration in the targeted tissue or cell.

The vitamin D prodrugs according to the present invention may be administered prophylactically, chronically, or acutely. For example, the vitamin D prodrugs may be administered prophylactically to inhibit the formation of diseases in the subject being treated. Specifically regarding cancer, the subject compounds may also be administered prophylactically to patients suffering a primary cancer to prevent the occurrence of metastatic cancers. In addition to the prevention of primary and metastatic cancers, chronic administration of the subject compounds will typically be indicated in treating recurring cancers. Acute administration of the subject compounds is indicated to treat, for example, aggressive cancers prior to surgical or radiological intervention.

The compounds, compositions, and method steps described herein can be used in any combination whether explicitly described or not.

All combinations of method steps as used herein can be performed in any order, unless otherwise specified or clearly implied to the contrary by the context in which the referenced combination is made.

As used herein, the singular forms "a," "an," and "the" include plural referents unless the content clearly dictates otherwise.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

The methods, compounds, and compositions of the present invention can comprise, consist of, or consist essentially of the essential elements and limitations described herein, as well as any additional or optional steps, ingredients, components, or limitations described herein or otherwise useful in the art.

It is understood that the invention is not confined to the particular construction and arrangement of parts herein illustrated and described, but embraces such modified forms thereof as come within the scope of the claims.

### EXAMPLES

### Example 1

In this example, the hypercalcemic effect of a preferred prodrug of the present invention, 25-β-glucuronide-1,25-dihydroxyvitamin D₃ (hereafter abbreviated as β-gluc-1,25-D₃) (see FIG. 3), was compared with that of 1,25-dihydroxyvitamin D₃ (hereinafter abbreviated as 1,25-D₃).

Adult, 350-g rats fed vitamin D-replete, 1%-calcium rat chow were treated with various doses of either 1,25-D₃ or β-gluc-1,25-D₃ by continuous subcutaneous infusion using mini-osmotic pumps (Model 2014, 14 day pumps, Alzet Corp). The pumps were filled aseptically with the appropriate dose of drug dissolved in propylene glycol. The pumps were surgically implanted under the skin on the dorsal neck and shoulder area aseptically. On the 12th day of infusion, rats were deeply anesthetized (isofluorane inhalation) and blood was collected by cardiac puncture for plasma calcium determination. The plasma calcium levels were determined by standard methods. The results of this experiment are shown in Table 2.

**Table 2. Effect of β-gluc-1,25-D₃ versus 1,25-D₃ on Plasma Calcium Levels**

| **Treatment** | **Dose (ng/day)** | **Plasma Calcium Mean ± SD** | **N (rats/ treatment)** | **Significantly greater than control? Duncan's Multiple Range Test** |
|---|---|---|---|---|
| Control (vehicle only) | 0 | 10.35 ± 0.50 | 11 | |
| 1,25-D₃ | 10 | 10.41 ± 0.27 | 12 | NO |
| 1,25-D₃ | 15 | 10.78 ± 0.26 | 6 | Yes |
| β-gluc-1,25-D₃ | 14 | 10.63 ± 0.20 | 6 | NO |
| β-gluc-1,25-D₃ | 70 | 10.71 ± 0.15 | 6 | NO |
| β-gluc-1,25-D₃ | 250 | 11.02 ± 0.49 | 6 | Yes |
| β-gluc-1,25-D₃ | 350 | 11.00 ± 0.25 | 6 | Yes |
| β-gluc-1,25-D₃ | 500 | 11.33 ± 0.38 | 6 | Yes |

The lowest dose of 1,25-D₃ inducing a statistically significant increase in plasma calcium levels was 15 ng/day. By contrast, the lowest dose of β-gluc-1,25-D₃ inducing a statistically significant increase in plasma calcium levels was 250 ng/day, with the next lower dose, 70 ng/day, inducing no increase in plasma calcium. Thus, the hypercalcemic effect of β-gluc-1,25-D₃ was at least 4-fold less than the native hormone (*i.e*., of from about 4-fold and about 18-fold). Very high doses of β-gluc-1,25-D₃ can be administered without causing severe hypercalcemia, as the 500-ng/day dose showed no evidence of a reduction in feed intake or other symptoms that might suggest compromised function from hypercalcemia.

### Example 2

To treat inflammatory bowel disease or other diseases of the lower intestine, a preferred vitamin D prodrug (whether derived from vitamin D₂, D₃, D₄, or D₅) bypasses the upper intestines and delivers the prodrug to the ileum and colon and only to those sites. Bacteria restricted to the lower intestine hydrolyse the prodrug and free the vitamin D-drug moiety comprising the active vitamin D drug within the colon and ileum. Here, the free vitamin D-drug stimulates vitamin D-mediated effects in the colon to the same or greater degree than in subjects treated directly with a vitamin D drug without a pro moiety. Because absorption of the cleaved vitamin D-drug moiety from the colon is not expected to be as efficient as in the small intestine, the systemic effects (increased plasma vitamin D concentration, bone resorption, and blood calcium) are less in subjects administered the glycosylated forms of the vitamin D drug orally or in their diet than those administered the non-glycosylated forms.

To determine β-glucuronidase activity capable of freeing a vitamin D-drug moiety from a conjugated β-glucuronide glycone in various parts of the small intestine, β-glucuronidase activity was tested in contents from intestinal subsections of 18 rats fed normal rat chow. The proximal 25 cm of duodenum/jejunum, the caudal 12 cm of ileum, and the cranial 12 cm of colon were removed. The contents from the lumen of each section were flushed with 3 ml water, collected, and pooled. Three-ml aliquots of intestinal contents were placed into tubes. To the tubes, either 2100 pg (3.53 pmoles) β-gluc-1,25-D₃ was added and incubated at 37°C for 0, 1, 3, or 6 hrs, or 150 pg (0.36 pmoles) of 1,25-D₃ was added and incubated for 0 or 6 hrs. The 1,25-D₃ served as a control to confirm the ability to extract and detect 1,25-D₃ in this material and to determine if any degradation of 1,25-D₃ would occur. Acetonitrile (3 ml) was added to each tube after incubation to end all enzymatic activity, after which the 1,25-D₃ was extracted. Tritiated 1,25-D₃ was added to each tube to assess extraction efficiency. The preparations were cleaned by HPLC, and the samples were analysed for 1,25-D₃ content by RIA (Heartland Assays, Ames, IA) (Hollis et al., J. Steroid Biochem. Mol. Biol. 2007 103(3-5):473-6). The results of this experiment are shown in Table 3.

**Table 3. β-Glucuronidase activity is confined to the lower intestine**

| **Lumen Contents** | **Incubation Time (hrs.)** | **Added Vitamin D Form** | **Added Amount (pg)** | **Measured Free 1,25-dihydroxyvitamin D (pg)** |
|---|---|---|---|---|
| Duodenu m | 0 | 1,25-D₃ | 150 | 96 |
| Duodenu m | 6 | 1,25-D₃ | 150 | 117 |
| Duodenu m | 0 | β-gluc-1,25-D₃ | 2100 | 29 |
| Duodenu m | 1 | β-gluc-1,25-D₃ | 2100 | 38 |
| Duodenu m | 3 | β-gluc-1,25-D₃ | 2100 | 63 |
| Duodenu m | 6 | β-gluc-1,25-D₃ | 2100 | 105 |
| Ileum | 0 | 1,25-D₃ | 150 | 129 |
| Ileum | 6 | 1,25-D₃ | 150 | 128 |
| Ileum | 0 | β-gluc-1,25-D₃ | 2100 | 39 |
| Ileum | 1 | β-gluc-1,25-D₃ | 2100 | 1323 |
| Ileum | 3 | β-gluc-1,25-D₃ | 2100 | 1127 |
| Ileum | 6 | β-gluc-1,25-D₃ | 2100 | 1342 |

The results shown in Table 3 show that the upper small intestine (duodenum) of rats does not contain sufficient β-glucuronidase activity to cause release of substantial amounts of 1,25-D₃ in the upper small intestine, even after 6 hrs. On the other hand, the lower small intestine (ileum) contains significant β-glucuronidase activity, and β-gluc-1,25-D₃ is likely to be rapidly cleaved upon entry to the ileum. Colon results were similar to the ileum (data not shown).

### Example 3

The results in the above example suggest that a vitamin D glycoside introduced to the alimentary canal would be selectively activated in the lower digestive tract such as the ileum and/or colon. A prominent action of 1,25-D₃ on its target tissues is induction of the mRNA for the Cyp24 enzyme. In this example, studies were conducted in mice to investigate the relative activity of β-gluc-1,25-D₃ and 1,25-D₃ on colon and duodenum, using Cyp24 expression as an indicator of action of the secosteroid on the tissues.

In a first study, 10-wk old, male C57BL/6 mice fed Teklad 2018, 1% calcium, vitamin D-replete diet (Madison, WI) *ad libitum* received a single equimolar dose (6, 12, 24, or 48 pmol) of either 1,25-D₃ or β-gluc-1,25-D₃ suspended in 50 µl peanut oil *per os* (4 mice/treatment). Mice were decapitated 6 hrs later following light anesthesia under inhaled halothane.

In a second study, similarly maintained mice were treated with a single 24 pmol dose of either 1,25-D₃ or β-gluc-1,25-D₃ suspended in 50 µl peanut oil *per os* (5 mice/treatment). Mice were then decapitated at 1, 3, 6 and 24 hrs after treatment.

For each study, blood from the cervical stump was collected into heparinized tubes, and plasma was harvested therefrom. The plasma samples were analysed for 1,25-D₃ content by RIA (Heartland Assays, Ames, IA) (Hollis et al. J Steroid Biochem Mol Biol. 2007, 103:473-476). β-gluc-1,25-D₃, being more water-soluble than 1,25-D₃ elutes with the methanol wash of the 0.5-g C₁₈OH SPE column (Varian, Lexington, MA) making it possible to measure only 1,25-D₃ in the samples.

In addition, a 1 cm section of duodenum (between 2 and 3 cm from the pylorus) and a 1 cm section of colon (between 2 and 3 cm from the cecum) were obtained from each mouse for mRNA analysis. Tissue samples were flushed with ice-cold phosphate-buffered saline and immediately homogenized in 1 ml of TRIzol® reagent (Invitrogen Corp., Carlsbad, CA). Samples were then kept frozen at-86°C prior to processing for RNA.

For processing RNA, each TRIzol® homogenate was thawed at room temperature and 500 µl placed in a clean microfuge tube, mixed thoroughly with 100 µl chloroform for 15 sec and then centrifuged at 12,000 X g for 15 min at 4°C. The upper aqueous phase was removed and mixed with 0.93 volumes of 75% EtOH. The mixture was then applied to an RNeasy spin column (Qiagen Inc., Germantown, MD) and processed as described by the manufacturer with the exception that an additional wash with 2M NaCl/2 mM EDTA (pH 4.0) was included (Das et al. J Vet Diagn Invest. 2009, ;21:771-778). RNA was eluted in 50 µl of water and the concentration obtained by UV spectrometry. One microgram of RNA was then used as a template for production of cDNA in a 20-µl reaction volume using random hexamers and Superscript III as described by the manufacturer (Invitrogen, Carlsbad, CA). Afterwards, samples were diluted to 100 µl final volume with TE buffer and stored at-20°C prior to PCR analysis.

Quantitative real-time RT-PCR was performed using a Stratagene Mx3005p cycler (Stratagene, La Jolla, CA) and PerfeCTa® SYBR® Green FastMix®, ROX reagent (Quanta Biosciences, Gaithersburg, MD). Amplification of target cDNAs was accomplished with the following primers:
Cyp24-For, 5' -CACACGCTGGCCTGGGACAC-3' (SEQ ID NO:1);
Cyp24-Rev, 5' -GGAGCTCCGTGACAGCAGCG-3' (SEQ ID NO:2);
GAPDH-For, 5' -GAAGGTCGGTGTGAACGGATTTGGC-3' (SEQ ID NO:3); and
GAPDH-Rev, 5' -TTGATGTTAGTGGGGTCTCGCTCCTG-3' (SEQ ID NO:4).

Aliquots (8.3 ng) of cDNA were amplified under the following conditions: 95°C for 30 sec, followed by 45 cycles of 95°C for 1 sec and 57°C for 30 sec. All reactions were performed in duplicate, with 4 or 5 animals per treatment and Cyp24 target gene expression was estimated using the ΔCT method relative to GAPDH expression as described previously (Giulietti et al. Methods 2001, 25:386-401). Oligonucleotides were obtained from Integrated DNA Technologies (Coralville, IA).

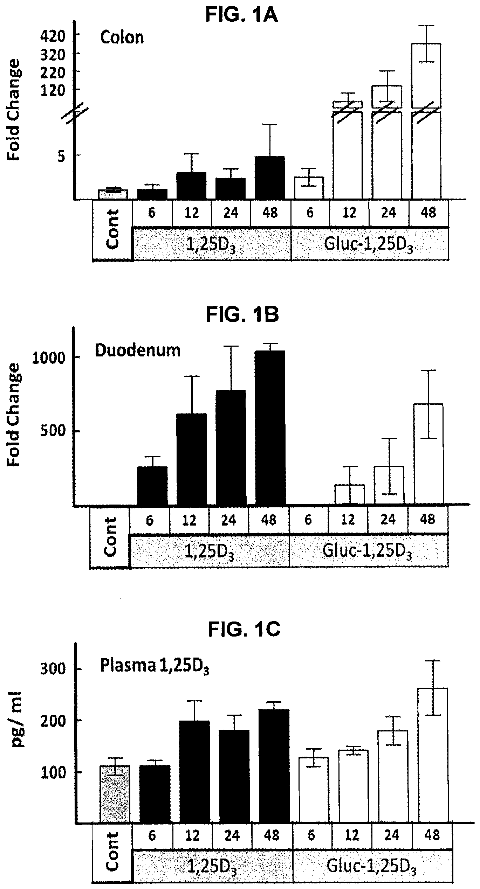

The effects of various doses (6, 12, 24 and 48 pmol) of β-gluc-1,25-D₃ and 1,25-D₃ on Cyp24 expression in the colon and duodenum relative to untreated mice at 6 hours after treatment are shown in FIGS 1A-C.

At the highest dose of 1,25-D₃ administered (48 pmol), there was approximately a 4.8 ± 4-fold increase in Cyp24 expression in the colon (FIG. 1A). The equimolar dose of β-gluc-1,25-D₃ caused over a 400 fold increase in colon Cyp24 expression (FIG. 1A). Even at the 12-pmol dose, β-gluc-1,25-D₃ was able to cause a 60-fold increase in Cyp24 expression in the colon (FIG. 1A), which was about 20 times greater than the response from the equimolar dose of 1,25-D₃.

As expected, 1,25-D₃ was able to strongly induce Cyp24 gene expression in the duodenums of the same mice 6 hours after oral dosing, with maximal induction (>1000-fold) occurring at the highest dose (48 pmol) evaluated (FIG. 1B). Though induction of Cyp24 gene expression was also observed in the duodenums of mice treated with the β-gluc-1,25-D₃ (FIG. 1B), it was consistently less effective than the analogous dose of 1,25-D₃.

Plasma 1,25-D₃ concentration was not significantly increased at the 6 hr time point by 6 pmol of either 1,25-D₃ or β-gluc-1,25-D₃ (FIG. 1C). Higher doses of either compound resulted in higher levels of 1,25-D₃ in the blood (FIG. 1C). The levels of 1,25-D₃ in the blood resulting from 1,25-D₃ versus β-gluc-1,25-D₃ at 6 hrs following treatment were comparable at each concentration (FIG. 1C). Plasma calcium concentrations were similar to control mouse plasma calcium concentrations in all treatment groups, which likely reflects the short time duration of the experiment.

The effects of 24 pmol of β-gluc-1,25-D₃ or 1,25-D₃ on Cyp24 expression in the colon and duodenum at various intervals after treatment are shown in FIGS. 2A-C.

The highest levels of expression of Cyp24 in both the colon and duodenum were observed at 3 or 6 hrs after treatment (FIGS. 2A and 2B, respectively). β-gluc-1,25-D₃ treatment caused Cyp24 in the colon to increase about 700-fold higher than in control mice at 6 hrs, whereas 1,25-D₃ was only able to increase colon Cyp24 about 5-fold (FIG. 2A).

In the duodenum, the relative effect of 1,25-D₃ and β-gluc-1,25-D₃ was reversed. Compared to control mice, 1,25-D₃ treatment steadily increased Cyp24 expression in the duodenum from the 1-hour time point (350-fold induction), to the 3-hour time point (1600-fold induction), and to the 6-hour time point (more than 2500 fold) (FIG. 2B). In contrast, the β-gluc-1,25-D₃ effects on Cyp24 peaked at 3 hrs in the duodenum with a 1300-fold increase in Cyp24 expression and had fallen to a 500-fold increase at 6 hours (FIG. 2B). The effects of both 1,25-D₃ and β-gluc-1,25-D₃ on Cyp24 gene expression in both tissues was similar to control mouse levels 24 hours after treatment (FIG. 2B).

Plasma concentrations of 1,25-D₃ peaked at 1280 pg/ml in 1 hr following the *per os* treatment with 24 pmol 1,25-D₃ (FIG. 2C). This is approximately a 14-fold increase over control mouse plasma 1,25-D₃ (FIG. 2C). In contrast, the average plasma 1,25-D₃ concentration in mice treated with 24 pmol β-gluc-1,25-D₃ peaked approximately 3 hrs after treatment at 325 pg/ml (FIG. 2C), a level that was only 3.5-fold greater than control levels. By 24 hrs after treatment plasma 1,25-D₃ concentrations in both 1,25-D₃ and β-gluc-1,25-D₃ treated mice were slightly below the concentration observed in control animals (FIG. 2C). Plasma calcium concentrations were similar to control mouse plasma calcium concentrations in all time points of both treatment groups.

Taken together these two studies demonstrate that oral administration of β-gluc-1,25-D₃ has a greater effect on colon tissue and a lesser effect on the duodenum than does the native hormone. Oral administration of β-gluc-1,25-D₃ also causes a much lower increase in plasma concentration of 1,25-D₃ than does the equimolar dose of 1,25-D₃. However, the time for each drug to cause peak levels of 1,25-D₃ in the blood differs. The highest plasma concentrations of 1,25-D₃ occur shortly after oral administration of 1,25-D₃, and the concentrations decline thereafter due to rapid metabolism of 1,25-D₃ in the mouse. However, there is a delay in the time that 1,25-D₃ concentrations peak in mice receiving β-gluc-1,25-D₃. This likely represents the time it takes for the compound to reach the ileum and to be converted to 1,25-D₃ prior to absorption. A result of this is that the plasma 1,25-D₃ peak with β-gluc-1,25-D₃ administration is much more blunted than with 1,25-D₃ administration, and the 1,25-D₃ concentrations over time are therefore more consistent.

With regard to efficacy in stimulating vitamin D-dependent effects, these results suggest that the majority of the administered 1,25-D₃ was absorbed before reaching the colon. This is consistent with the data showing that administered 1,25-D₃ induced vitamin D-dependent effects in the duodenum but had very little effect in the colon, as well as the data showing the early spike of plasma 1,25-D₃ shortly after 1,25-D₃ administration. By contrast, the data suggest that β-gluc-1,25-D₃ was capable of reaching the colon without being substantially absorbed in the duodenum, thereby producing vitamin D-dependent effects in the colon.

### Example 4

Previous studies have suggested use of 1,25-D₃ for treatment of inflammatory bowel disease (IBD) (Froicu et al. BMC Immunology 2007 8:5). However, the dose required to improve intestinal inflammation in the mouse model of inflammatory disease (50 ng) results in hypercalcemia (Froicu et al.). The risk of hypercalcemia has prevented the use of 1,25-dihydroxyvitamin D for treatment of IBD in humans.

In this example, β-gluc-1,25-D₃ compounds, alone or in combination with 25-β-glucuronide-25-hydroxyvitamin D₃ compounds (hereinafter β-gluc-25-D₃) were incorporated into the diet of mice and compared to equimolar 1,25-D₃ with respect to therapeutic effects on IBD and induction of calcemia. A standard mouse model of IBD comprising administering dextran sodium sulfate (DSS) to induce inflammation of the lower colon was used to study these effects. Vitamin D treatments were first initiated for 4 days. After 4 days, mice were fed DSS-water for 7 days, allowed one day to recover, and sacrificed. Colon length, plasma calcium, fecal blood score, and body weight were assayed. Colon length is a standard marker of intestinal inflammation in the literature, with shortened colon length being an indicator of inflammation. A 1-cm section of the mid-colon was removed, fixed in formalin, and stained with hematoxylin & eosin for microscopic histopathologic evaluation by a veterinary pathologist blinded to the treatments. Each tissue section received a score from 0 to 4 reflecting absence of lesion to severe, extensive lesion for each of three criteria: (1) the degree of erosion/ulceration of colon mucosa; (2) the degree of infiltration of the tissues by inflammatory cells; and (3) the degree of submucosal edema. The sum of the score of each of these criteria constitutes the histopathological score for the tissue with a zero score representing normal tissue based on all criteria, and the worst possible outcome being a score of 12. The results of this experiment are shown in Table 4.

Referring to Table 4, when compared to DSS-treated controls, colon length was significantly (p<0.05) improved by the combination treatment of 70 ng/day β-gluc-1,25-D₃ with 5000 ng/day β-gluc-25-D₃. This combination treatment also improved fecal blood scores and maintained body weight of subjects better than any other treatment without leading to a physiologically significant increase in plasma Ca²⁺. The combination treatment with β-gluc-1,25-D₃ at 14 ng/day was also favorable to fecal blood scores and colon length, but the improvement failed to reach statistical significance. By contrast, 50 ng/day 1,25-D₃ was less effective than the 70-ng/day β-gluc-1,25-D₃ plus 5000 ng/day β-gluc-25-D₃ combination treatment in improving inflammation and was accompanied by a physiologically significant increase in hypercalcemia. While not as effective as the combination treatment the β-gluc-1,25-D₃ alone at 70 ng/day was numerically better than 1,25-D₃ at reducing inflammation and was not accompanied by a physiologically significant increase in hypercalcemia.

All the treatments except for the 350-ng/day β-gluc-1,25-D₃ and the 5000-ng/day β-gluc-25-D₃ treatments statistically improved (P<0.05) histologic pathology scores over the DSS controls. None of the treatments removed all evidence of pathology induced by the DSS. There were no statistical differences among the effective treatments in histopathological scores, although the native 1,25-D₃ at 50 ng/day was numerically the best. However, this treatment, unlike the β-gluc-1,25-D₃ alone at 70 ng/day or in combination with 5000 ng/day β-gluc-25-D₃, also caused considerable hypercalcemia.

**Table 4. Effect of β-Glucuronide Compounds on Colon Inflammation**

| **Treatment (9 mice)** | **Dose (ng/ day)** | **Colon Length (cm)** | **Plasma Calcium (mg/dl)** | **Fecal Blood Score^{a}** | **Final Body Weight (g)** | **Histopathology score (0-12)^{b}** |
|---|---|---|---|---|---|---|
| Control (no DSS) | | 6.89±0.37 ^{c} | 9.54±0.11 | 0±0^{c} | 23.77±0.52 ^{c} | 0.55±0.18 ^{c} |
| DSS | | 5.22±0.20 | 8.44±0.36^{d} | 0.89±0.34 | 20.91±0.77 | 9.33±0.62 |
| DSS + 1,25-D₃ | 10 | 5.52±0.20 | 9.68±0.19 | 0.33±0.12 ^{c} | 21.39±0.82 | 7.44±0.50 |
| DSS + 1,25-D₃ | 50 | 5.74±0.17 | 11.58±0.1 8^{d} | 0.22±0.12 ^{c} | 20.82±0.43 | 5.89±0.54 ^{c} |
| DSS + β-gluc-1,25-D₃ | 14 | 5.78±0.09 | 10.25±0.3 3 | 0.55±0.26 | 21.91±0.49 | 6.66±0.53 ^{c} |
| DSS + β-gluc-1,25-D₃ | 70 | 5.41±0.15 | 10.12±0.3 0 | 0.22±0.08 ^{c} | 21.00±0.68 | 6.33±0.74 ^{c} |
| DSS + β-gluc-1,25-D₃ | 350 | 4.72±0.15 | 10.72±0.3 3^{d} | 0.88±0.43 | 19.62±0.69 | 8.33±0.64 |
| DSS + β-gluc-25-D₃ | 5000 | 5.50±0.20 | 8.68±0.27^{d} | 1.05±0.34 | 21.16±0.53 | 8.22±0.72 |
| DSS + β-gluc-1,25-D₃ + β-gluc-25-D₃ | 14 | 5.63±0.16 | 9.35±0.32 | 0.50±0.17 | 22.01±0.60 | 7.56±0.47 |
| | 5000 | | | | | |
| DSS + β-gluc-1,25-D₃ + β-gluc-25-D₃ | 70 | 6.22±0.19 ^{c} | 10.26±0.2 2 | 0.11±0.11 ^{c} | 22.24±0.60 ^{c} | 6.78±0.98 ^{c} |
| | 5000 | | | | | |

| | | | | | | |
|---|---|---|---|---|---|---|
| ^{a}Fecal Blood Score: 0 = no blood to 3 = multiple blood spots in cage ^{b}Histopathology colon lesion score: 0 = no lesions; 12 = severe erosion, hemorrhage, and submucosal edema ^{c}significantly different from DSS Only mice (p<0.05) ^{d}Significantly different from Control (No DSS) mice (P<0.05). | | | | | | |

Consistent with the data presented in Example 3, this example indicates that β-glucuronide-vitamin D glycosides are effective in treating IBD, a disease of the lower intestine, without stimulating hypercalcemia. It also demonstrates that the administration of a competitive inhibitor of the 24-hydroxylase (5000 ng/day β-gluc-25-D₃) potentiates the action of the 1,25-D₃ aglycone, since the 5000 ng/day β-gluc-25-D₃ was essentially ineffective by itself.

### Example 5

This example shows that a 1,25-D₃ glycoside reduces proliferation of cancer cells in tissue culture.

LNCaP cells (ATCC # CRL-1740) are malignant prostate cancer cells originally obtained from a lymph node of a 50-yr-old man whose prostatic cancer had metastasized to the lymph node. LNCaP cells were grown in tissue culture using RPMI-1640 media with 10% fetal bovine serum. Wells of two 48-well tissue culture plates were seeded with 5000 LNCaP cells/well. The cells were allowed to adhere and establish residence for 24 hrs, after which they were left untreated or treated with 1,25-D₃ or β-gluc-1,25-D₃ for the next six days. Half the media was replaced with fresh media with treatments on the third day of the treatment period. By day 6 of treatment, wells of control untreated cells were approximately 60% confluent. The live cell numbers in each well at the end of treatment were assessed using the "CELLTITER BLUE"-brand assay kit (Promega Corp., Madison, WI). The assay is based on the ability of living cells to convert a redox dye (resazurin) into a fluorescent end product (resorufin). The relative proliferation index was defined with respect to control cell proliferation given a value of 100. The results of this experiment are shown in Table 5.

These results confirm studies demonstrating that the native hormone 1,25-D₃ is a potent inhibitor of LNCaP prostatic cancer cell growth in vitro (see Peehl et al. Cancer Res. 1994 54(3): 805-10). It also suggests that the cancer cell line is capable of cleaving the vitamin D glycoside, β-gluc-1,25-D₃, to the active aglycone, which then decreases proliferation of the cells. These results show that cancer cells express the appropriate glycosidases to cleave β-gluc-1,25-D₃ into its active form.

**Table 5. Effect of β-gluc-1,25-D₃ on LNCaP Cancer Cell Proliferation**

| **LNCaP Cell Treatment (N=12 wells/treatment)** | **Proliferation index (Mean ± SEM)** |
|---|---|
| Control (untreated) | 100 ± 3.6 |
| 20 nM 1,25-D₃ | 69.7 ± 8.4 |
| 100 nM 1,25-D₃ | 63.8 ± 7.3 |
| 20 nM β-gluc-1,25-D₃ | 95.4 ± 2.4 |
| 100 nM β-gluc-1,25-D₃ | 73.1 ± 9.5 |

A similar study was performed using a second human-derived prostatic cancer cell line known as DU-145 (ATCC# HTB-81). This cell line is also known to respond to 1,25-D₃ but is less sensitive than LNCaP cells (Feldman et al., Adv. Exp. Med. Biol. 1995 375:53-63). The DU-145 cells were propagated in Eagle's Minimum Essential Medium with 10% fetal bovine serum. The cells were treated in culture for just 4 days, with half the media replaced with fresh media on day 3 of treatment. Cells were 40% confluent at the end of treatment. The results of this experiment are shown in Table 6.

In DU-145 cells, β-gluc-1,25-D₃ is as active as the native hormone in reducing cell proliferation, though anti-proliferative activity of both compounds on DU 145 cells is reduced compared to activity against LNCaP cells.

**Table 6. Effect of β-gluc-1,25-D₃ on DU-145 Cancer Cell Proliferation**

| **DU-145 Cell Treatment (N=6 wells/treatment)** | **Proliferation index (Mean ± SEM)** |
|---|---|
| Control (untreated) | 100 ± 1.5 |
| 20 nM 1,25-D₃ | 94.7 ± 1.7 |
| 100 nM 1,25-D₃ | 89.3 ± 2.4 |
| 20 nM β-gluc-1,25-D₃ | 90.3 ± 2.2 |
| 100 nM β-gluc-1,25-D₃ | 88.7 ± 2.8 |

### Example 6

This examples tests the effect of subcutaneously administered β-gluc-1,25-D₃ on progression of mammary tumour growth in mice.

4T1 tumour cells, originally isolated from a BALB/cfC3H mouse mammary gland, were obtained from American Tissue Culture Collection (Manassas, VA). The tumour growth and metastatic spread of 4T1 cells in BALB/c mice very closely mimic human breast cancer. This syngeneic tumour graft is an animal model for stage IV human breast cancer (Pulaski et al. Cancer Res. 1998, 58:1486-1493). The cells were grown in RPMI-1640 media supplemented with 10% fetal bovine serum. When cell growth reached about 70% confluency, the cells were lifted from the flasks with standard removing medium and rinsed with 0.25% trypsin, 0.53-mM EDTA solution (trypsin-EDTA solution). This solution was removed and an additional 1 to 2 ml of trypsin-EDTA solution was added. The flask was allowed to sit at 37.0°C until the cells detached. Fresh culture medium was added, and the cells were aspirated for enumeration using a hemocytometer. RPMI-1640 and Matrigel (BD Biosciences, Bedford, MA) solutions were prepared and maintained at 4°C to keep the Matrigel liquid. Cells were brought up in RPMI-1640 and dispensed into a test tube so that there were 500,000 cells/100 µl of 50:50 RPMI-1640:Matrigel (BD Biosciences, Bedford, MA) and gently agitated so that the solution could then be used to fill tuberculin syringes that were also maintained on cold packs at 4-6°C until injected into the mice. Fifty female BALB/c mice fed Teklad 2018 diet (1% Calcium and vitamin D replete) were injected subcutaneously in the right paralumbar region with 500,000 4T1 cells. Sixteen days after implantation of the cells, tumours approximately 0.5-0.7 cm in diameter (as measured with calipers) formed under the skin of many of the mice. Mice were grouped into nine pairs with each pair having similarly sized tumours. One mouse from each pair was randomly assigned to either the treatment or control group. The treatment group (N=9) received 280 ng/day β-gluc-1,25-D₃ suspended in 50 µl sterile propylene glycol and delivered by daily subcutaneous injection. The control group (N=9) received 50 µl propylene glycol injected subcutaneously daily. After eight days of treatment, the mice were euthanized, as some mice in the control group had tumours that reached the limit considered humane. Blood was collected from each animal and the tumour mass was excised from each animal and weighed.

The mean ± SEM (N=9/group) tumour weight was 3.05 ± 0.26 g in control animals vs. 2.01 ± 0.36 g in animals treated with β-gluc-1,25-D₃. This demonstrated that the β-gluc-1,25-D₃ had a significant anti-proliferative effect on the growth of 4T1 tumour cells in mice bearing the syngeneic graft (P = 0.034). Plasma calcium of control mice was 8.48 ± 0.10 mg/dl, which was slightly below expected and may reflect cachexia from the tumour masses. Plasma calcium of β-gluc-1,25-D₃ treated mice was 10.12 ± 0.13 mg/dl, which was slightly above expected (control mice of inflammatory bowel disease mice experiment described above averaged 9.54 ± 0.11 mg/dl). However, this degree of hypercalcemia does not constitute severe or symptomatic hypercalcemia.

This example demonstrates that the vitamin D glycosides of the present invention are effective in treating tumours without inducing symptomatic hypercalcemia.

According to a further aspect of the present invention, there is provided a pharmaceutical composition comprising a therapeutically effective and non-severe-symptomatic-hypercalcemia-inducing amount of a vitamin D prodrug, wherein the vitamin D prodrug comprises a vitamin D-drug moiety and a pro moiety, and wherein the pro moiety is selected from the group consisting of a glycone moiety and a sulfate moiety, for use in treating a vitamin D-sensitive disease selected from the group consisting of a hyperproliferative, autoimmune, and infectious disease without inducing severe symptomatic hypercalcemia.

The vitamin D-drug moiety may comprise a vitamin D receptor agonist.

The vitamin D-drug moiety may comprise a substrate for autocrine production of a 1,25 dihydroxyvitamin D compound.

The pro moiety may comprise glucuronic acid.

The pharmaceutical composition may further comprise a non-severe-symptomatic-hypercalcemia-inducing amount of a second vitamin D prodrug comprising a vitamin D-drug moiety and a glycone moiety, wherein the vitamin D-drug moiety of the second vitamin D prodrug is a 24-hydroxylase inhibitor

The amount of the second vitamin D prodrug may potentiate a therapeutic effect of the vitamin D prodrug.

The vitamin D-drug moiety of the second vitamin D prodrug may lack a hydroxyl group at a C-1 position on the vitamin D-drug moiety and may comprise a hydroxyl group at a position selected from the group consisting of a C-24 position and a C-25 position on the vitamin D-drug moiety.

The vitamin D-drug moiety of the second vitamin D prodrug may be selected from the group consisting of 25-hydroxyvitamin D₂, 24,25-dihydroxyvitamin D₂, 25-hydroxyvitamin D₃, 24,25-dihydroxyvitamin D₃, 25-hydroxyvitamin D₄, 24,25-dihydroxyvitamin D₄, 25-hydroxyvitamin D₅, and 24,25-dihydroxyvitamin D₅.

The pharmaceutical composition may be for use in treating an autoimmune disease selected from the group consisting of inflammatory bowel disease.

The pharmaceutical composition may be for use in treating a hyperproliferative disease selected from the group consisting of cancers of the intestine, colon,

The pharmaceutical composition may be for use in increasing a level of free vitamin D-drug moiety in plasma to an increased amount, wherein the increased amount is no more than about 14 times an amount of baseline plasma vitamin D levels.

The pharmaceutical composition may be for use in maintaining the free vitamin D-drug moiety in plasma to within about ±70% of the increased amount over a 3-hour period after administration of the vitamin D prodrug.

The pharmaceutical composition may be for use in treating a vitamin D-sensitive intestinal disease.

The pharmaceutical composition of may be for use in treating an autoimmune disease selected from the group consisting of irritable bowel syndrome, Crohn's disease, and celiac disease.

The pharmaceutical composition may be for use in treating inflammatory bowel disease.

The pharmaceutical composition may be for use in treating a vitamin D-sensitive intestinal disease selected from the group consisting of ulcerative colitis and pseudomembranous colitis.

The pharmaceutical composition may be for use in treating colorectal cancer.

The pharmaceutical composition may be for use in selectively treating the vitamin D-sensitive intestinal disease in the lower intestine, comprising cleaving the vitamin D prodrug in the lower intestine.

The pharmaceutical composition may be for use in maintaining a level of free vitamin D-drug moiety in plasma to less than about 14 times an amount of baseline plasma vitamin D levels.

## Claims

1. A pharmaceutical composition comprising a therapeutically effective and non-severe-symptomatic-hypercalcemia-inducing amount of a first compound and a second compound;
wherein the first compound is a vitamin D prodrug comprising a vitamin D-drug moiety and a pro moiety, wherein the pro moiety is selected from the group consisting of a glycone moiety and a sulfate moiety, wherein the vitamin D prodrug has the structure of Formula (I): 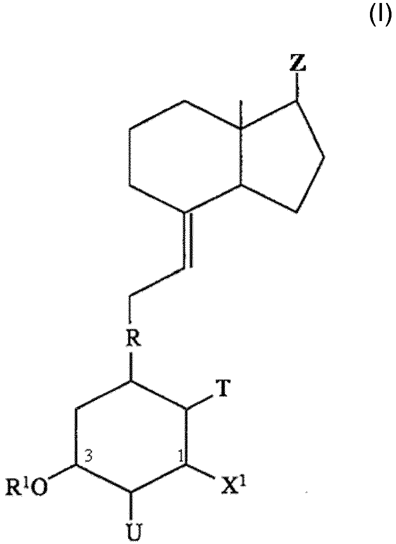
wherein T is hydrogen or a =CH₂ group;
X¹ is selected from the group consisting of -OH and -OR¹;
U is hydrogen, C₁-C₆ alkenyl, C₁-C₆ alkyl, -OH, or -O-(C₂-C₄ alkyl)-OH;
R is a double bond or an epoxy group;
R¹ is hydrogen, -SO₃, or a straight- or branched-chain glycone moiety comprising 1-20 glycone units, or R¹ is an orthoester glycoside moiety of Formula (II): 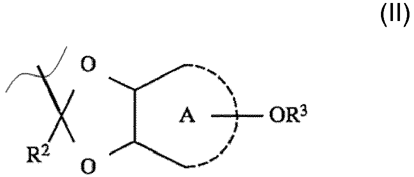
wherein A represents a glycofuranosyl or glycopyranosyl ring;
R² is hydrogen, lower alkyl, aralkyl, or aryl, with the proviso that aryl is phenyl or phenyl substituted by chloro, fluoro, bromo, iodo, lower C₁-C₄ alkyl, C₁-C₄ alkoxy; or naphthyl; and
R³ is hydrogen, -SO₃, or a straight- or branched-chain glycone moiety comprising 1-20 glycone units; and
Z is a hydrogen or a saturated or unsaturated, substituted or unsubstituted, straight-chain or branched C₁-C₁₈ hydrocarbon group having a formula as represented by Formula (III): 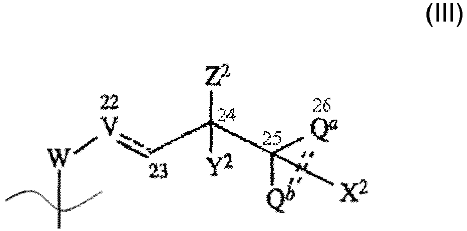
wherein the bond between C-22 and C-23 is a single or double bond;
Y² is hydrogen, fluorine, C₁-C₆ alkyl, -OH, or -OR¹;
Z² is hydrogen, fluorine, C₁-C₆ alkyl, -OH, or -OR¹;
Q*^{a}* is -CF₃ or -CH₂X²;
Q*^{b}* is -CF₃ or -CH₃;
X² is hydrogen, -OH, or -OR¹;
W is -CH-CH₃ or -O-;
V is CH₂, CH, or -O-, wherein W and V are not both -O-; and
"= = =" is either a single bond between Q^{a} and Q^{b} or a hydrogen atom on both Q^{a} and Q^{b}, wherein when "= = =" is a single bond, X² is H;
wherein at least one of the R¹ comprises at least one glycone moiety or at least one -SO₃ moiety;
wherein the second compound comprises a first moiety and a second moiety, wherein the first moiety is selected from the group consisting of a glycone moiety and a sulfate moiety, and wherein the second moiety is a 24-hydroxylase inhibitor selected from the group consisting of 25-hydroxyvitamin D₂, 24,25-dihydroxyvitamin D₂, 25-hydroxyvitamin D₃, 24,25-dihydroxyvitamin D₃, 25-hydroxyvitamin D₄, 24,25-dihydroxyvitamin D₄, 25-hydroxyvitamin D₅, and 24,25-dihydroxyvitamin D₅,
for use in selectively treating a vitamin D-sensitive disease in the intestine.

2. A pharmaceutical composition for use as claimed in claim 1, wherein the vitamin D-drug moiety of the vitamin D prodrug comprises 1,25-dihydroxyvitamin D.

3. A pharmaceutical composition for use as claimed in claim 1, wherein the vitamin D-drug moiety of the vitamin D prodrug comprises a substrate for autocrine production of a 1,25-dihydroxyvitamin D compound.

4. A pharmaceutical composition for use as claimed in claim 1, 2 or 3, wherein the pro moiety of the vitamin D prodrug or the first moiety of the second compound comprises a glycone moiety.

5. A pharmaceutical composition for use as claimed in any preceding claim, wherein the pro moiety of the vitamin D prodrug or the first moiety of the second compound comprises glucuronic acid.

6. A pharmaceutical composition for use as claimed in claim 1, 2 or 3, wherein the pro moiety of the vitamin D prodrug or the first moiety of the second compound comprises a sulfate moiety.

7. A pharmaceutical composition for use as claimed in any preceding claim, wherein the amount of the second compound competitively inhibits vitamin D 24-hydroxylase.

8. A pharmaceutical composition for use as claimed in any preceding claim, wherein the second moiety of the second compound is selected from the group consisting of 25-hydroxyvitamin D₂, 24,25-dihydroxyvitamin D₂, 25-hydroxyvitamin D₄, 24,25-dihydroxyvitamin D₄, 25-hydroxyvitamin D₅, and 24,25-dihydroxyvitamin D₅.

9. A pharmaceutical composition as claimed in any preceding claim, for use in treating a cancer of the intestine.

10. A pharmaceutical composition as claimed in any preceding claim, for use in treating a cancer of the colon.

11. A pharmaceutical composition as claimed in any preceding claim, for use in selectively treating a vitamin D-sensitive disease in the lower intestine.

12. A pharmaceutical composition as claimed in any preceding claim, for use in selectively treating a vitamin D-sensitive intestinal disease.

13. A pharmaceutical composition as claimed in any preceding claim, for use in treating inflammatory bowel disease.

## Patentansprüche

1. Pharmazeutische Zusammensetzung, umfassend eine therapeutisch wirksame und nicht schwerwiegende symptomatische Hyperkalzämie induzierende Menge einer ersten Verbindung und eine zweite Verbindung; wobei die erste Verbindung ein Vitamin-D-Prodrug ist, umfassend einen Vitamin-D-Arzneimittelrest und einen Pro-Rest, wobei der Pro-Rest ausgewählt ist aus der Gruppe bestehend aus einem Glykonrest und einem Sulfatrest, wobei das Vitamin-D-Prodrug die Struktur der Formel (I) hat: 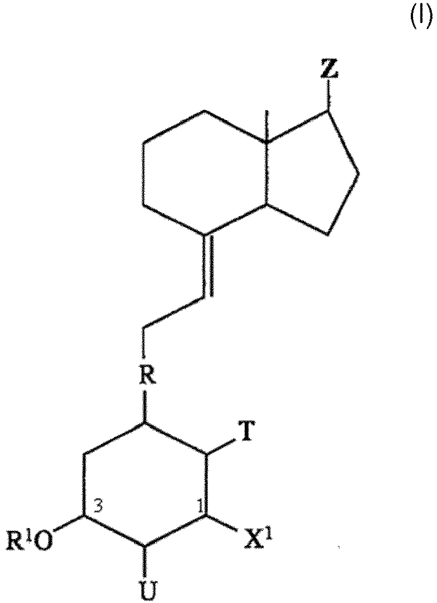
wobei T Wasserstoff oder eine =CH₂-Gruppe ist;
X¹ ausgewählt ist aus der Gruppe bestehend aus -OH und -OR¹;
U Wasserstoff, C₁-C₆-Alkenyl, C₁-C₆-Alkyl, -OH oder -O-(C₂-C₄-Alkyl)-OH ist;
R eine Doppelbindung oder eine Epoxygruppe ist;
R¹ Wasserstoff, -SO₃ oder ein gradkettiger oder verzweigtkettiger Glykonrest ist, welcher 1 - 20 Glykoneinheiten umfasst, oder R¹ ein Glykosidorthoesterrest der Formel (II) ist: 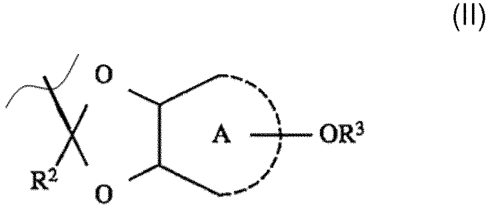
wobei A einen Glykofuranosyl- oder Glykopyranosylring darstellt;
R² Wasserstoff, Niedrigalkyl, Aralkyl oder Aryl ist, mit der Maßgabe, dass Aryl Phenyl oder mit Chlor, Fluor, Brom Iod, C₁-C₄-Niedrigalkyl, C₁-C₄-Alkoxy substituiertes Phenyl ist; oder Naphthyl; und
R³ Wasserstoff, -SO₃ oder ein geradkettiger oder verzweigtkettiger Glykonrest ist, welcher 1 - 20 Glykoneinheiten umfasst; und
Z ein Wasserstoff oder eine gesättigte oder ungesättigte, substituierte oder unsubstituierte, geradkettige oder verzweigte C₁-C₁₈-Kohlenwasserstoffgruppe ist, welche eine wie in Formel (III) dargestellte Formel besitzt: 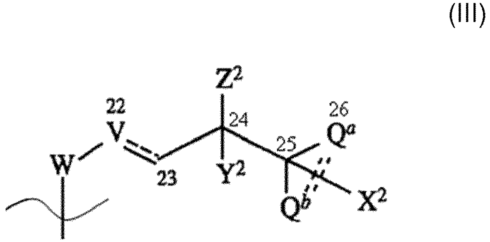
wobei die Bindung zwischen C-22 und C-23 eine Einfach- oder Doppelbindung ist;
Y² Wasserstoff, Fluor, C₁-C₆-Alkyl, -OH oder -OR¹ ist;
Z² Wasserstoff, Fluor, C₁-C₆-Alkyl, -OH oder -OR¹ ist;
Q^{a} -CF₃ oder -CH₂X² ist;
Q^{b} -CF₃ oder -CH₃ ist;
X² Wasserstoff, -OH oder -OR¹ ist;
W -CH-CH₃ oder -O- ist;
V CH₂, CH oder -O- ist, wobei W und V nicht beide -O- sind; und
"= = =" entweder eine Einfachbindung zwischen Q^{a} und Q^{b} oder ein Wasserstoffatom an beiden von Q^{a} und Q^{b} ist, wobei, wenn "= = =" eine Einfachbindung ist, X² H ist;
wobei wenigstens einer von R¹ wenigstens einen Glykonrest oder wenigstens einen -SO₃-Rest umfasst; wobei die zweite Verbindung einen ersten Rest und einen zweiten Rest umfasst, wobei der erste Rest ausgewählt ist aus der Gruppe bestehend aus einem Glykonrest und einem Sulfatrest, und wobei der zweite Rest ein 24-Hydroxylaseinhibitor ist, ausgewählt aus der Gruppe bestehend aus 25-Hydroxyvitamin-D₂, 24,25-Dihydroxyvitamin-D₂, 25-Hydroxyvitamin-D₃, 24,25-Dihydroxyvitamin-D₃, 25-Hydroxyvitamin-D₄, 24,25-Dihydroxyvitamin-D₄, 25-Hydroxyvitamin-D₅ und 24,25-Dihydroxyvitamin-D₅,
zur Verwendung bei der selektiven Behandlung einer Vitamin-D-empfindlichen Erkrankung im Darm.

2. Pharmazeutische Zusammensetzung zur Verwendung gemäß Anspruch 1, wobei der Vitamin-D-Arzneimittelrest des Vitamin-D-Prodrugs 1,25-Dihydroxyvitamin-D umfasst.

3. Pharmazeutische Zusammensetzung zur Verwendung gemäß Anspruch 1, wobei der Vitamin-D-Arzneimittelrest des Vitamin-D-Prodrugs ein Substrat zur autokrinen Bildung einer 1,25-Dihydroxyvitamin-D-Verbindung umfasst.

4. Pharmazeutische Zusammensetzung zur Verwendung gemäß Anspruch 1, 2 oder 3, wobei der Pro-Rest des Vitamin-D-Prodrugs oder der erste Rest der zweiten Verbindung einen Glykonrest umfasst.

5. Pharmazeutische Zusammensetzung zur Verwendung gemäß irgendeinem voranstehenden Anspruch, wobei der Pro-Rest des Vitamin-D-Prodrugs oder der erste Rest der zweiten Verbindung Glucuronsäure umfasst.

6. Pharmazeutische Zusammensetzung zur Verwendung gemäß Anspruch 1, 2 oder 3, wobei der Pro-Rest des Vitamin-D-Prodrugs oder der erste Rest der zweiten Verbindung einen Sulfatrest umfasst.

7. Pharmazeutische Zusammensetzung zur Verwendung gemäß irgendeinem voranstehenden Anspruch, wobei die Menge der zweiten Verbindung Vitamin-D-24-Hydroxylase konkurrierend inhibiert.

8. Pharmazeutische Zusammensetzung zur Verwendung gemäß irgendeinem voranstehenden Anspruch, wobei der zweite Rest der zweiten Verbindung ausgewählt ist aus der Gruppe bestehend aus 25-Hydroxyvitamin-D₂, 24,25-Dihydroxyvitamin-D₂, 25-Hydroxyvitamin-D₄, 24,25-Dihydroxyvitamin-D₄, 25-Hydroxyvitamin-D₅ und 24,25-Dihydroxyvitamin-D₅.

9. Pharmazeutische Zusammensetzung, gemäß irgendeinem voranstehenden Anspruch, zur Verwendung bei der Behandlung von Darmkrebs.

10. Pharmazeutische Zusammensetzung, gemäß irgendeinem voranstehenden Anspruch, zur Verwendung bei der Behandlung von Dickdarmkrebs.

11. Pharmazeutische Zusammensetzung, gemäß irgendeinem voranstehenden Anspruch, zur Verwendung bei der selektiven Behandlung einer Vitamin-D-empfindlichen Erkrankung im unteren Darmbereich.

12. Pharmazeutische Zusammensetzung, gemäß irgendeinem voranstehenden Anspruch, zur Verwendung bei der selektiven Behandlung einer Vitamin-D-sensitiven Darmerkrankung.

13. Pharmazeutische Zusammensetzung, gemäß irgendeinem voranstehenden Anspruch, zur Verwendung bei der Behandlung einer entzündlichen Erkrankung des Gedärms.

## Revendications

1. Composition pharmaceutique comprenant une quantité thérapeutiquement efficace et induisant une hypercalcémie symptomatique non sévère d'un premier composé et d'un second composé ;
dans laquelle le premier composé est un promédicament vitamine D comprenant une fraction médicament vitamine D et une pro fraction, la pro fraction étant choisie dans le groupe consistant en une fraction glycone et une fraction sulfate, le promédicament vitamine D ayant la structure de Formule (I) : 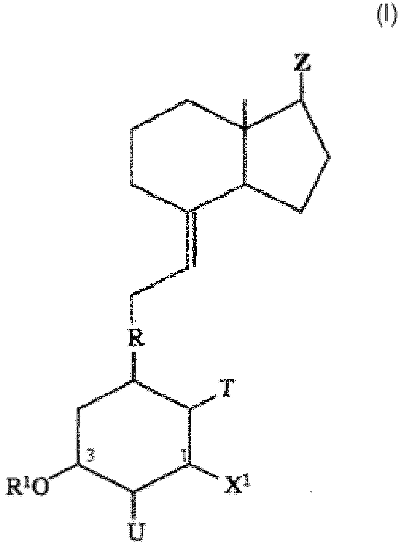
dans laquelle
T représente hydrogène ou un groupe =CH₂ ;
X¹ est choisi dans le groupe consistant en -OH et -OR¹ ;
U représente hydrogène, alcényle en C₁-C₆, alkyle en C₁-C₆, -OH ou -O-(alkyl en C₂-C₄)-OH ;
R est une double liaison ou un group époxy ;
R¹ représente hydrogène, -SO₃ ou une fraction glycone à chaîne droite ou ramifiée, comprenant 1-20 unités glycone, ou R¹ est une fraction orthoester glycoside de Formule (II) : 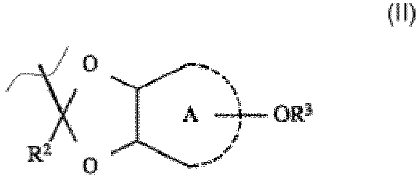
dans laquelle
A représente un noyau glycofuranosyle ou glycopyranosyle ;
R² représente hydrogène, alkyle inférieur, aralkyle ou aryle, à la condition qu'aryle représente phényle ou phényle substitué par chloro, fluoro, bromo, iodo, alkyle en C₁-C₄ inférieur, alcoxy en C₁-C₄ ; ou naphtyle ; et
R³ représente hydrogène, -SO₃ ou une fraction glycone à chaîne droite ou ramifiée, comprenant 1-20 unités glycone ; et
Z représente un hydrogène ou un groupe hydrocarboné en C₁-C₈ à chaîne droite ou ramifié, substitué ou non substitué, saturé ou insaturé, ayant une formule telle que représentée par la Formule (III) : 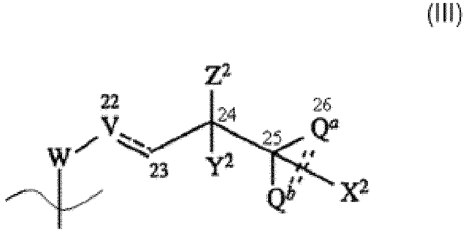
dans laquelle
la liaison entre C-22 et C-23 est une simple liaison ou une double liaison ;
Y² représente hydrogène, fluor, alkyle en C₁-C₆, -OH ou -OR¹ ;
Z² représente hydrogène, fluor, alkyle en C₁-C₆, -OH ou -OR¹ ;
Q^{a} représente -CF₃ ou -CH₂X² ;
Q^{b} représente -CF₃ ou -CH₃ ;
X² représente hydrogène, -OH ou -OR¹ ;
W représente -CH-CH₃ ou -O- ;
V représente CH₂, CH ou -O-, où W et V ne sont pas tous deux -O- ; et
« = = = » représente soit une simple liaison entre Q^{a} et Q^{b} soit un atome d'hydrogène sur à la fois Q^{a} et Q^{b}, où lorsque « = = = » est une simple liaison, X² représente H ;
où au moins l'un des R¹ comprend au moins une fraction glycone ou au moins une fraction -SO₃ ;
où le second composé comprend une première fraction et une seconde fraction, la première fraction étant choisie dans le groupe consistant en une fraction glycone et une fraction sulfate, et la seconde fraction étant un inhibiteur de 24-hydroxylase choisi dans le groupe consistant en 25-hydroxyvitamine D₂, 24,25-dihydroxyvitamine D₂, 25-hydroxyvitamine D₃, 24,25-dihydroxyvitamine D₃, 25-hydroxyvitamine D₄, 24,25-dihydroxyvitamine D₄, 25-hydroxyvitamine D₅ et 24,25-dihydroxyvitamine D₅,
pour une utilisation dans le traitement sélectif d'une maladie sensible à la vitamine D dans l'intestin.

2. Composition pharmaceutique pour une utilisation selon la revendication 1, dans laquelle la fraction médicament vitamine D du promédicament vitamine D comprend 1,25-dihydroxyvitamine D.

3. Composition pharmaceutique pour une utilisation selon la revendication 1, dans laquelle la fraction médicament vitamine D du promédicament vitamine D comprend un substrat pour la production autocrine d'un composé 1,25-dihydroxyvitamine D.

4. Composition pharmaceutique pour une utilisation selon l'une des revendications 1, 2 ou 3, dans laquelle la pro fraction du promédicament vitamine D ou la première fraction du second composé comprend une fraction glycone.

5. Composition pharmaceutique pour une utilisation selon l'une quelconque des revendications précédentes, dans laquelle la pro fraction du promédicament vitamine D ou la première fraction du second composé comprend de l'acide glucuronique.

6. Composition pharmaceutique pour une utilisation selon l'une des revendications 1, 2 ou 3, dans laquelle la fraction pro du promédicament vitamine D ou la première fraction du second composé comprend une fraction sulfate.

7. Composition pharmaceutique pour une utilisation selon l'une quelconque des revendications précédentes, dans laquelle la quantité du second composé inhibe de façon compétitive la vitamine D 24-hydroxylase.

8. Composition pharmaceutique pour une utilisation selon l'une quelconque des revendications précédentes, dans laquelle seconde fraction du second composé est choisi dans le groupe consistant en 25-hydroxyvitamine D₂, 24,25-dihydroxyvitamine D₂, 25-hydroxyvitamine D₄, 24,25- dihydroxyvitamine D₄, 25-hydroxyvitamine D₅ et 24,25-dihydroxyvitamine D₅.

9. Composition pharmaceutique selon l'une quelconque des revendications précédentes, pour une utilisation dans le traitement d'un cancer de l'intestin.

10. Composition pharmaceutique selon l'une quelconque des revendications précédentes, pour une utilisation dans le traitement d'un cancer du côlon.

11. Composition pharmaceutique selon l'une quelconque des revendications précédentes, pour une utilisation dans le traitement sélectif d'une maladie sensible à la vitamine D dans le gros intestin.

12. Composition pharmaceutique selon l'une quelconque des revendications précédentes, pour une utilisation dans le traitement sélectif d'une maladie intestinale sensible à la vitamine D.

13. Composition pharmaceutique selon l'une quelconque des revendications précédentes, pour une utilisation dans le traitement d'une maladie intestinale inflammatoire.
